# EUROPEAN PATENT SPECIFICATION

(11) **EP 3 538 214 B1**
(45) Date of publication and mention of the grant of the patent: **05.07.2023**
(21) Application number: 17801269.6
(22) Date of filing: 08.11.2017
(51) Int. Cl.: A61N 1/32, A61B 18/14, A61B 18/00, H03K 3/57, A61B 18/12, A61N 1/40

(54) **LOW-VOLTAGE IMPEDANCE CHECK PULSE GENERATOR**
IMPULSGENERATOR ZUR IMPEDANZPRÜFUNG MIT NIEDRIGER SPANNUNG
GÉNÉRATEUR D'IMPULSIONS DE VÉRIFICATION D'IMPÉDANCE BASSE TENSION

(30) Priority: 09.11.2016 US 201615347728
(43) Date of publication of application: 18.09.2019
(73) Proprietor: Pulse Biosciences, Inc., Hayward, CA 94545 (US)
(72) Inventor: ATHOS, Brian G., San Francisco, California 94116 (US); DANITZ, David J., San Jose, California 95136 (US); KREIS, Mark P., San Francisco, California 94112 (US); UECKER, Darrin R., San Mateo, California 94401 (US)
(74) Representative: IP Sextant s.r.l.
(86) International application number: PCT/US2017/060654
(87) International publication number: WO 2018/089506

(56) References cited:
- US-A1- 2002 193 833
- US-A1- 2003 204 161
- US-A1- 2007 066 957
- US-A1- 2008 077 189
- US-A1- 2015 032 100
- US-A1- 2015 272 657

## Description

### Field of Technology

The present application generally relates to electrical pulse techniques including circuits and systems for generating electric pulses, including the use of an energy-accumulating element discharged through a load by a relatively low voltage transistor and for controlling the discharge. Specifically, the pulse techniques are used for generating variable duration nanosecond pulsed electric fields (nsPEF) for electrotherapy.

### BACKGROUND

Surgical excision of a tumor can result in an infection and leave a scar. Furthermore, if there are more tumors, every cancerous tumor should be identified and individually excised by a surgeon. This can be time consuming and expensive, not to mention uncomfortable for patients. As used herein a patient includes a subject receiving therapeutic treatment or experimental treatment.

Cancerous tumors that are internal to a patient may be especially difficult to remove, let alone detect and treat. Many patients' lives are turned upside down by the discovery of cancer in their bodies, sometimes which have formed relatively large tumors before being detected.

A "nanosecond pulsed electric field," sometimes abbreviated as nsPEF, includes an electric field with a sub-microsecond pulse width of, for example, between 0.1 nanoseconds (ns) and 1000 nanoseconds, or as otherwise known in the art. It is sometimes referred to as sub-microsecond pulsed electric field. NsPEFs often have high peak voltages, such as 10 kilovolts per centimeter (kV/cm), 20 kV/cm, to 500 kV/cm. Treatment of biological cells with nsPEF technology often uses a multitude of periodic pulses at a frequency ranging from 0.1 per second (Hz) to 10,000 Hz.

NsPEFs have been found to trigger apoptosis in cancerous tumors. Selective treatment of such tumors with nsPEFs can induce apoptosis within the tumor cells without substantially affecting normal cells in the surrounding tissue due to its non-thermal nature.

An example of nsPEF applied to biological cells is shown and described in U.S. Patent No. 6,326,177 (to Schoenbach et al.). Further, Background art is described in the documents US2002/193833, US2003/204161 and US2007/066957 all relating to electroporation pulsed electric fields systems.

The use of nsPEF for the treatment of tumors is a relatively new field. The nsPEF pulses are generated from a charged pulse generator, and there exists a need for a device with better control over pulse generator charge state for safe and effective studies and treatments of cancer in human subjects.

### BRIEF SUMMARY

Generally, a nanosecond pulsed electric field (nsPEF) generator is disclosed that incorporates one or more enegy storage devices used to generate relatively high voltage or relatively low voltage nsPEF pulses. The nsPEF generator may include a discharge circuit which can be selectively used to discharge the energy storage devices from a high voltage to a low voltage so that low voltage pulses may be applied. Alternatively, the nsPEF generator may have a separate low voltage power supply used for generating the low voltage pulses.

In some embodiments, the high voltage nsPEF pulses may be used for therapeutic treatment of tissue. In some embodiments, the low voltage nsPEF pulses are used to test the setup of the nsPEF generator system. For example, once the system is set up, one or more low voltage pulses may be applied to the load. The current generated by or causing the pulses is monitored to determine a load impedance. The determined load impedance is compared with an expected impedance of the load, for example, based on an identification of the material or tissue type of the load. A determined impedance outside of an expected range, for example, indicates a setup problem, a load problem, an electrode problem, or another system problem.

While not claimed, described herein for completeness and better understanding is a method of testing a pulse generator circuit. The method includes charging the pulse generator circuit to a first charge voltage, with the pulse generator circuit, delivering a first voltage pulse to a load through an electrode, and determining an impedance of the load with the first voltage pulse. The method also includes comparing the impedance with an expected impedance and determining whether to deliver a second voltage pulse to the load based at least in part on a result of the comparison, where at least one of the first and second voltage pulses is therapeutic to the load.

In some aspects of the method, determining the impedance of the load includes measuring a current, where the current is delivered to the load during the delivery of the first voltage pulse, determining a voltage of the delivered first voltage pulse, and calculating the impedance based on a voltage level of the first voltage pulse and on the measured current.

In some embodiments of the method, the voltage level of the first pulse is less than a voltage level of the second pulse.

In some aspects, the method also includes determining the expected impedance based on an identification of the load.

In some aspects of the method, the expected impedance includes an impedance range.

In some aspects of the method, determining whether to deliver the second voltage pulse includes determining to deliver the second pulse to the load as a result of the calculated impedance being one of the following: 1) less than a threshold difference from the expected impedance, 2) within an expected impedance range, where the expected impedance includes the expected impedance range, 3) less than a maximum threshold, and 4) greater than a minimum threshold.

In some aspects of the method, the voltage level of the first pulse is greater than a voltage level of the second pulse.

In some aspects of the method, determining whether to deliver the second voltage pulse includes determining to deliver the second pulse to the load as a result of the calculated impedance being one of: 1) greater than a threshold difference from the expected impedance, 2) outside an expected impedance range, where the expected impedance includes the expected impedance range, 3) greater than a maximum threshold, and 4) less than a minimum threshold.

In some aspects of the method, the method comprises delivering the second voltage pulse, which includes charging or discharging the pulse generator circuit to a second charge voltage.

In some aspects of the method, delivering the second voltage pulse includes changing a state of a switch so as to electrically disconnect the load from a first voltage pulse source and to connect the load to a second voltage pulse source.

In some aspects of the method, the first and second voltage pulses are therapeutic to the load.

In some aspects, the method also includes displaying a graphical indication based on the comparison.

In some aspects, the method also includes delivering the second voltage pulse to the load, determining a second impedance of the load, comparing the second impedance with the expected impedance, and determining to stop delivering therapeutic voltage pulses in response to the comparison of the second impedance with the expected impedance.

Currently claimed invention relates to a therapeutic nsPEF pulse generator system according to independent claims 1 and 15. Further advantageous embodiments are described below and in dependent claims 2-14 and 16-19.

In some examples of the system, the voltage level of the first pulse is less than a voltage level of the second pulse.

In some examples of the system, the controller is further configured to determine the expected impedance based on an identification of the load.

In some examples of the system, the expected impedance includes an impedance range.

In some examples of the system, the controller is configured to cause delivery of the second pulse to the load as a result of the calculated impedance being one of the following: 1) less than a threshold difference from the expected impedance, 2) within an expected impedance range, where the expected impedance includes the expected impedance range, 3) less than a maximum threshold, and 4) greater than a minimum threshold.

In some examples of the system, the voltage level of the first pulse is greater than a voltage level of the second pulse.

In some examples of the system, the controller is configured to cause delivery of the second pulse to the load as a result of the calculated impedance being one of the following: 1) greater than a threshold difference from the expected impedance, 2) outside an expected impedance range, where the expected impedance includes the expected impedance range, 3) greater than a maximum threshold, and 4) less than a minimum threshold.

In some examples of the system, causing delivery of the second voltage pulse includes causing charging or discharging the pulse generator circuit to a second charge voltage.

In some examples of the system, causing delivery the second voltage pulse includes causing the change of a state of a switch so as to electrically disconnect the load from a first voltage pulse source and to connect the load to a second voltage pulse source.

In some examples of the system, the first and second voltage pulses are therapeutic to the load.

In some examples of the system, the system is configured to display a graphical indication based on the comparison.

In some examples of the system, the controller is further configured to cause delivery of the second voltage pulse, determine a second impedance of the load, compare the second impedance with the expected impedance, and determine to stop delivering therapeutic voltage pulses in response to the comparison of the second impedance with the expected impedance.

Also not claimed but described herein for completeness another method of using a pulse generator circuit, such as a therapeutic pulse generator circuit. The method includes, with the pulse generator circuit, delivering a first voltage pulse to a load through an electrode, and determining a first impedance of the load based partly on the delivered first voltage pulse. The method also includes, with the pulse generator circuit, delivering a second voltage pulse to a load through an electrode, and determining a second impedance of the load based partly on the delivered second voltage pulse. The method also includes comparing the first impedance with the second impedance, and at least in part as a result of the comparison: 1) stopping delivery of voltage pulses to the load, 2) confirming continued delivery of voltage pulses of similar treatment pulse parameters, or 3) delivering a next voltage pulse to the load, where the next voltage pulse has an electrical parameter which is different from a corresponding electrical parameter of the first and second voltage pulses.

In some aspects of the method, the first and second voltage pulses are therapeutic pulses.

In some aspects of the method, the first and second voltage pulses are test pulses.

In some aspects of the method, delivery of voltage pulses to the load is stopped as a result of a difference between the first and second impedances being greater or less than a threshold or outside of a threshold range.

In some aspects of the method, the voltage pulse having the different electrical parameter is delivered as a result of the comparison indicating a deviation from an impedance profile being greater than a threshold.

In some examples, the electrical parameter is one of a voltage, a frequency, a duration, and a voltage shape. Other features and advantages of the devices of the present disclosure will become apparent from the following detailed description of one or more implementations when read in view of the accompanying figures.

### BRIEF DESCRIPTION OF THE DRAWINGS

FIG. 1 illustrates a nanosecond pulse generator apparatus in accordance with an embodiment.
FIG. 2 illustrates a pulse profile for both voltage and current in accordance with an embodiment.
FIG. 3 illustrates a perspective view of a seven-needle electrode in accordance with an embodiment.
FIG. 4 illustrates a perspective view of a two-pole electrode in accordance with an embodiment.
FIG. 5 is an electrical schematic of a pulse generator in accordance with an embodiment.
FIG. 6A is a schematic illustrating the pulse generator shown in FIG. 5 during charge mode.
FIG. 6B is a schematic illustrating the pulse generator shown in FIG. 5 during discharge mode.
FIG. 7 is an electrical schematic of an assembly of pulse generator circuits.
FIG. 8 is an electrical schematic of one of the pulse generator circuits shown in FIG. 7.
FIG. 9 is an electrical schematic of one of the pulse generator stages shown in FIG. 8.
FIG. 10 is an electrical schematic of one of the switch drivers shown in FIG. 9.
FIG. 11 is an electrical schematic of an alternative switch element.
FIG. 12 is a waveform diagram illustrating the operation of a transformer and a control voltage to a MOSFET gate.
FIG. 13 is an alternative electrical schematic of a pulse generator shown in FIG. 1.
FIG. 14 is an alternative electrical schematic of a pulse generator shown in FIG. 1.
FIG. 15 is an electrical schematic of an embodiment of one of the pulse generator circuits shown in FIG. 7 having a discharge circuit.
FIG. 16 is an electrical schematic of an embodiment of one of the pulse generator stages shown in FIG. 15 having a discharge circuit stage.
FIG. 17 is a schematic illustration of an embodiment of a discharge circuit stage used in the pulse generator stage of FIG. 16.
FIG. 18 is an electrical schematic of an embodiment a pulse generator circuit having a discharge circuit.
FIG. 19 is an illustration of an embodiment of a peak voltage source.
FIG. 20 is an electrical schematic of an alternative pulse generator circuit.
FIG. 21 is a block diagram of a nsPEF treatment system.
FIG. 22 is a table of expected impedances for certain treatment loads.
FIG. 23 is a flowchart diagram illustrating a method, not part of the claimed invention, of testing a pulse generator system setup status.
FIG. 24 is a flowchart diagram illustrating a method, not part of the claimed invention, of testing a pulse generator system setup status.
FIG. 25 is a flowchart diagram illustrating a method, not part of the claimed invention, of testing a pulse generator system setup status.
FIG. 26 is a flowchart diagram illustrating a method, not part of the claimed invention, of using a pulse generator system.

### DETAILED DESCRIPTION

It has been shown that nsPEF treatments can be used to cause cancerous tumor cells to undergo apoptosis, a programmed cell death. Tests have shown that tumors can shrink to nonexistence after treatment. No drugs may be necessary. It has also been shown that the subject's immune system may be stimulated to attack all similar tumor cells, including those of tumors that are not within the nsPEF-treated tumor.

A "tumor" includes any neoplasm or abnormal, unwanted growth of tissue on or within a subject, or as otherwise known in the art. A tumor can include a collection of one or more cells exhibiting abnormal growth. There are many types of tumors. A malignant tumor is cancerous, a pre-malignant tumor is precancerous, and a benign tumor is noncancerous. Examples of tumors include a benign prostatic hyperplasia (BPH), uterine fibroid, pancreatic carcinoma, liver carcinoma, kidney carcinoma, colon carcinoma, pre-basal cell carcinoma, and tissue associated with Barrett's esophagus.

A "disease" includes any abnormal condition in or on a subject that is associated with abnormal, uncontrolled growths of tissue, including those that are cancerous, precancerous, and benign, or other diseases as known in the art.

"Apoptosis" of a tumor or cell includes an orderly, programmed cell death, or as otherwise known in the art.

"Immunogenic apoptosis" of a tumor or cell includes a programmed cell death that is followed by an immune system response, or as otherwise known in the art. The immune system response is thought to be engaged when the apoptotic cells express calreticulin or another antigen on their surfaces, which stimulates dendritic cells to engulf, consume, or otherwise commit phagocytosis of the targeted cells leading to the consequent activation of a specific T cell response against the target tumor or cell.

Pulse lengths of between 10 and 900 nanoseconds for nsPEF have been particularly studied to be effective in stimulating an immune response. Pulse lengths of about 100 nanoseconds are of particular interest in that they are long enough to carry sufficient energy to be effective at low pulse numbers but short enough to be effective in the manner desired.

A time of "about" a certain number of nanoseconds includes times within a tolerance of ±1%, 2%, 3%, 4%, 5%, 7.5%, 10%, 15%, 20%, 25% or other percentages, or fixed tolerances, such as ±0.1, ±0.2, ±0.3, ±0.4, ±0.5, ±0.7, ±1.0, ±2.0, ±3.0, ±4.0 ±5.0, ±7.0, ±10, ±15, ±20, ±25, ±30, ±40, ±50, ±75 ns, or other tolerances as acceptable in the art in conformance with the effectivity of the time period.

Immune system biomarkers can be measured before and/or after nsPEF treatment in order to confirm that the immune response has been triggered in a patient. Further, nsPEF treatment can be paired with a CD47-blocking antibody treatment to better train CD8+T cells (i.e., cytotoxic T cells) for attacking the cancer.

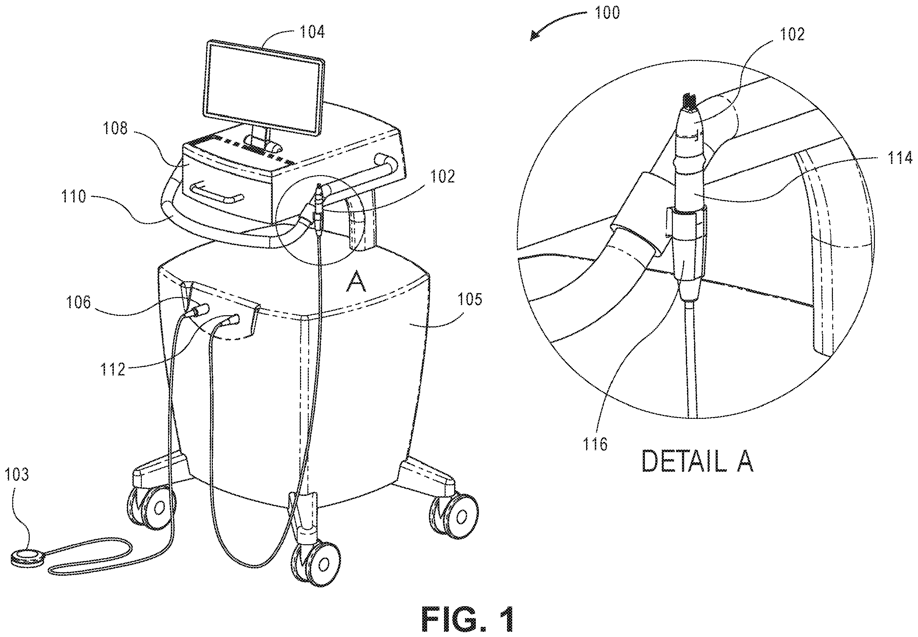

FIG. 1 illustrates a nanosecond pulse generator system in accordance with an embodiment. NsPEF system 100 includes electrode 102, footswitch 103, and interface 104. Footswitch 103 is connected to housing 105 and the electronic components therein through connector 106. Electrode 102 is connected to housing 105 and the electronic components therein through high voltage connector 112. NsPEF system 100 also includes a handle 110 and storage drawer 108. As shown in DETAIL A portion of FIG. 1, nsPEF system 100 also includes holster 116, which is configured to hold electrode 102 at its handle portion 114.

A human operator inputs a number of pulses, amplitude, pulse duration, and frequency information, for example, into a numeric keypad or a touch screen of interface 104. In some embodiments, the pulse width can be varied. A microcontroller sends signals to pulse control elements within nsPEF system 100. In some embodiments, fiber optic cables allow control signaling while also electrically isolating the contents of the metal cabinet with nsPEF generation system 100, the high voltage circuit, from the outside. In order to further isolate the system, system 100 may be battery powered instead of from a wall outlet.

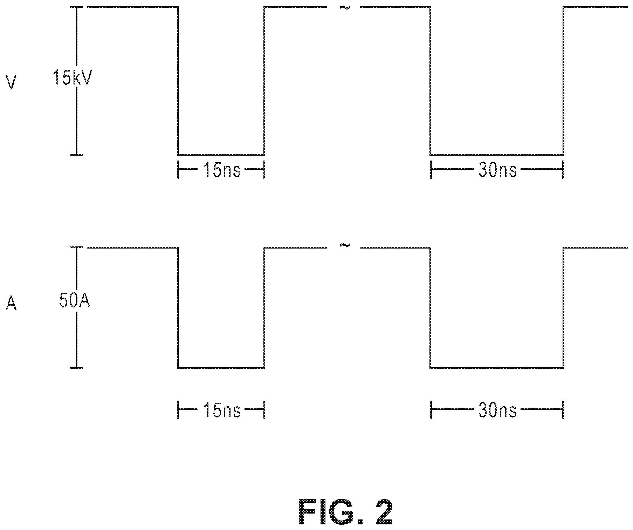

FIG. 2 illustrates a pulse profile for both voltage and current in accordance with an embodiment. Output from the nsPEF system 100 with voltage on the top of the figure and current on the bottom for a first and second pulses. The first pulse has an amplitude of about 15 kV, a current of about 50 A, and a duration of about 15 ns. The second pulse has an amplitude of about 15 kV, a current of about 50 A, and a duration of about 30 ns. If such a pulse had been delivered on suction electrodes having 4 mm between the plates, the pulse generator would have delivered a pulse of about 50 A and 37.5 kV/cm. Given a voltage, current depends heavily on the electrode type and tissue resistance.

While FIG. 2 illustrates a specific example, other pulse profiles may also be generated. For example, in some embodiments, rise and/or fall times for pulses may be less than 20 ns, about 20 ns, about 25 ns, about 30 ns, about 40 ns, about 50 ns, about 60 ns, about 75 ns, or greater than 75 ns. In some embodiments, the pulse voltage may be less than 5 kV, about 5 kV, about 10 kV, about 15 kV, about 20 kV, about 25 kV, about 30 kV, or greater than 30 kV. In some embodiments, the current may be less than 10 A, about 10 A, about 25 A, about 40 A, about 50 A, about 60 A, about 75 A, about 100 A, about 125 A, about 150 A, about 175 A, about 200 A, or more than 200 A. In some embodiments, the pulse duration may be less than 10 ns, about 10 ns, about 15 ns, about 20 ns, about 25 ns, about 30 ns, about 40 ns, about 50 ns, about 60 ns, about 75 ns, about 100 ns, about 125 ns, about 150 ns, about 175 ns, about 200 ns, about 300 ns, about 400 ns, about 500 ns, about 750 ns, about 1 µs, about 2 µs, about 3 µs, about 4 µs, about 5 µs, or greater than 5 µs.

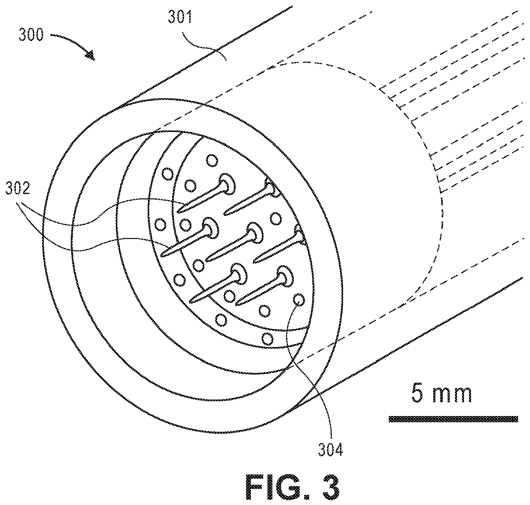

FIG. 3 illustrates a perspective view of a seven-needle suction electrode in accordance with an embodiment. In electrode 300, sheath 301 surrounds seven sharp electrodes 302 with an broad opening at a distal end. When the open end is placed against a tumor, air is evacuated from the resulting chamber through vacuum holes 304 to draw the entire tumor or a portion thereof into the chamber. The tumor is drawn so that one or more of the electrodes preferably penetrates the tumor. Sharp ends of the electrodes are configured to pierce the tumor. The center electrode may be at one polarity, and the outer six electrodes may be at the opposite polarity. Nanopulses electric fields can then be precisely applied to the tumor using nsPEF system 100 (see FIG. 1).

The electrodes can be apposed, one of each positive and negative pair of electrodes on one side of a tumor and the other electrode of the pair on an opposing side of the tumor. Opposing sides of a tumor can include areas outside or within a tumor, such as if a needle electrode pierces a portion of the tumor.

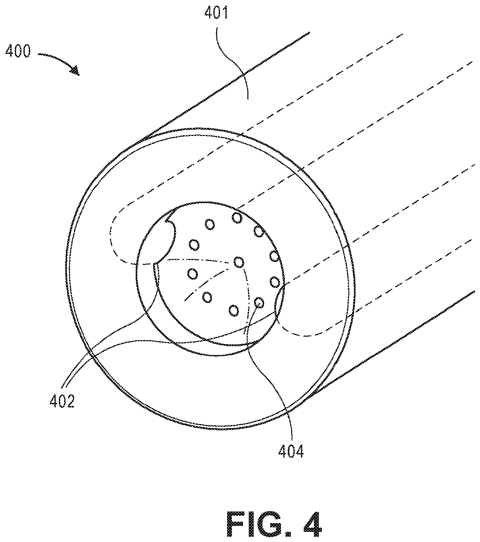

FIGS. 4 illustrates a two-pole suction electrode in accordance with an embodiment. In electrode device 400, sheath 401 surrounds two broad electrodes 402 on opposite sides of a chamber. When air is evacuated through vacuum holes 404 and a tumor is pulled within the chamber, the opposing electrodes apply nsPEF pulses to the tumor.

The nature of the electrode used mainly depends upon the shape of the tumor. Its physical size and stiffness can also be taken into account in selection of a particular electrode type.

U.S. Patent No. 8,688,227 B2 (to Nuccitelli et al.) discloses other suction electrode-based medical instruments and systems for therapeutic electrotherapy.

If there are multiple tumors in a subject, a surgeon can select a single tumor to treat based on the tumor's compatibility with electrodes. For example, a tumor that is adjacent to a stomach wall may be more easily accessible than one adjacent a spine or the brain. Because a nsPEF pulse is preferably applied so that the electric field transits through as much tumor mass as possible while minimizing the mass of non-tumor cells that are affected, a clear path to two opposed 'poles' of a tumor may also be a selection criterion.

For tumors on or just underneath the skin of subject, needle electrodes can be used percutaneously. For locations deeper within a subject, a retractable electrode can fit into a gastroscope, bronchoscope, colonoscope, or other endoscope or laparoscope. For example, a tumor in a patient's colon can be accessed and treated using an electrode within a colonoscope. For tumors within the body, electrodes can be used in open surgery, laparoscopic surgery, or through other minimally invasive surgical approaches.

Barrett's esophagus, in which portions of tissue lining a patient's esophagus are damaged, may be treated using an electrode placed on an inflatable balloon.

Embodiments of nanosecond pulsed power generators produce electric pulses in the range of single nanoseconds to single microseconds. The pulses are created by rapid release of energy stored in, for example, a capacitive or inductive energy reservoir to a load in a period that is generally much shorter than the charging time of the energy reservoir.

Conventional capacitive-type pulsed generators include pulse forming networks, which provide fixed pulse duration and impedance. With prior knowledge of a load's resistance, a pulse forming network with impedance that matches the load can be used. But for broader applications, especially when the load resistance is unknown, it is desirable to have a pulse generator with a flexibility of impedance matching and variation of pulse duration. Such flexibility can be implemented by switching a capacitor with a controllable switch. In this case, the capacitor can be regarded as a "voltage source" and can adapt to various load resistance. The switched pulse amplitude can then have the same voltage as the voltage of the capacitor. The pulse width is accordingly determined by the switch "on" time.

The selection of switches in nanosecond pulse generators is limited because of the high voltages, high currents, and fast switching times involved.

Spark gap switches, typically used in pulsed power technology, are capable of switching high voltages and conducting high currents. But they can only be turned on, and stopping the current flow in the middle of conduction is impossible. Besides spark gaps, other types of high voltage, high power switches are available, such as: magnetic switches, vacuum switches, gas filled tubes (Thyratrons for example), and certain high-voltage semiconductor switches.

Magnetic switches, relying on the saturation of magnetic core, change from high impedance to low impedance in the circuit. They can be turned on above a certain current threshold but will not be turned off until all the current is depleted by the load.

Vacuum switches are a good option for high voltage and high repletion rate operation, but similar to magnetic switches, they also can be only turned on, but cannot be turned off at a predetermined time.

Some types of high-voltage semi-conductor switches may also be considered. Thyristors and insulated gate bipolar transistors (IGBTs) may, in some embodiments be used. However, the turn-on times of Thyristors and IGBTs limit their usefulness.

Metal-oxide-semiconductor field-effect transistors (MOSFETs) have insufficient maximum drain to source voltage ratings (e.g. < 1kV) and insufficient maximum drain to source current ratings (e.g. < 50A) to be used in conventional pulse generator architectures to produce the voltage and current necessary for the applications discussed herein. If they were used, a large number of stages would be needed in order to produce high-amplitude output voltages. However, in conventional Marx generator architectures with a large number of stages, the Marx generator goes into an underdamped mode instead of a critically damped mode, resulting in loss in overshoot. As a result, the overall voltage efficiency decreases. For example, a voltage efficiency of a Marx generator may be 80% at 5 stages but decrease to 50% at 20 stages.

Furthermore, as the number of stages is increased, the impedance of the Marx generator also increases. This reduces the total energy deliverable to the load. This is particularly unfavorable for driving low impedance loads and long pulses.

In addition, the charging losses in the charging resistors also increases with the increased number of stages. As a result, such Marx generators are unsuitable for high repetition rate operation.

Therefore, in order to produce high voltage pulses, simply increasing the number of stages will cause a series of problems, including low efficiency, high impedance, etc. Because there is a tradeoff between the number of the stages and the actual output voltage, using conventional Marx generators cannot produce high voltage pulses which are sufficient for the applications discussed herein.

Some embodiments of this disclosure include a tunable, high voltage, nanosecond pulse generator. The switches may be power MOSFETs, which may, for example, be rated for a voltage of 1 kV and current of up to 30A. In some embodiments, the switches power MOSFETs rated for a voltage of 1 kV and current of up to continuous 90A and more than 200A peak. Voltage is scaled up by a Marx-switch stack hybrid circuit. In each Marx generator stage, a particularly configured stack of MOSFETs is used. As a result, the charging voltage for each stage is greater than the rated maximum for a single switch.

A technical advantage of the configuration is that the overall output voltage can be increased with just a few stages (e.g. <=5). As a result, the problems discussed above with Marx generators having a large number of stages are avoided and high efficiency, low impedance, and large variability in the pulse duration can be achieved.

Such an architecture also allows much easier control as only one trigger circuit may be needed for each stage. One additional benefit is that the pulse generator has low impedance, so it will be able to drive various loads with high current and extended pulse duration. The scaling up of the current is implemented by combining multiple Marx-switch stack circuits in parallel. The pulse duration is controlled by the closing and opening of the switch stack switches.

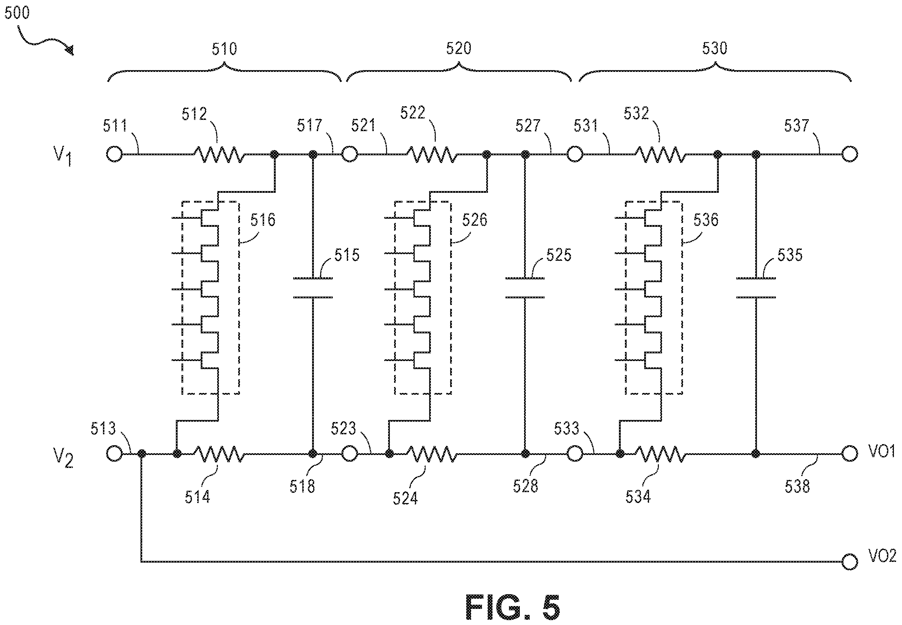

FIG. 5 illustrates a pulse generator circuit 500 which may be used inside nsPEF system 100 of FIG. 1. Pulse generator circuit 500 illustrates a panel comprising a Marx generator switched by three switch stacks. The nsPEF system can have a single pulse generator circuit panel. In some embodiments, a nsPEF system includes multiple panels in parallel.

Circuit 500 includes three stages - 510, 520, and 530. In some embodiments, another number of stages is used. For example, in some embodiments, 2, 4, 5, 6, 7, 8, 9, or 10 stages are used. Stage 510 includes resistors 512 and 514, capacitor 515, and switch stack 516. Likewise, stage 520 includes resistors 522 and 524, capacitor 525, and switch stack 526, and stage 530 includes resistors 532 and 534, capacitor 535, and switch stack 536. Each of these elements have structure and functionality which is similar to the corresponding elements of stage 510.

Stage 510 has first and second input voltage input terminals 511 and 513 and first and second voltage output terminals 517 and 518. Stage 520 has first and second input voltage input terminals 521 and 523, and first and second voltage output terminals 527 and 528. Stage 530 has first and second input voltage input terminals 531 and 533, and first and second voltage output terminals 537 and 538.

The first and second voltage input terminals 511 and 513 of stage 510 are respectively connected to first and second power supply input terminals V1 and V2. The first and second voltage output terminals 517 and 518 of stage 510 are respectively connected to the first and second voltage input terminals 521 and 523 of stage 520. The first and second voltage output terminals 527 and 528 of stage 520 are respectively connected to the first and second voltage input terminals 531 and 533 of stage 530. The second voltage output terminal 538 of stage 530 and second voltage input terminal 513 of stage 510 are respectively connected to first and second power output terminals VO1 and VO2.

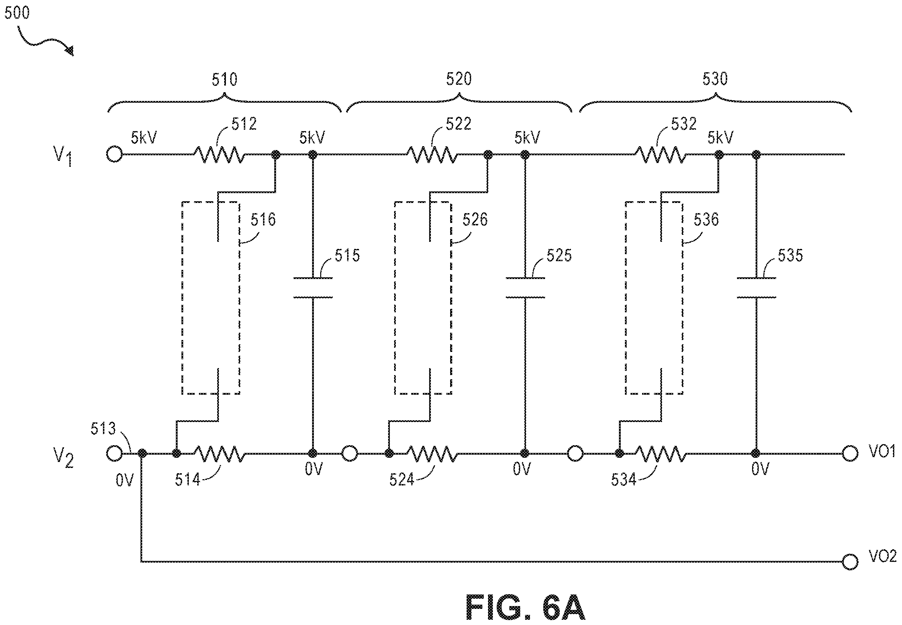

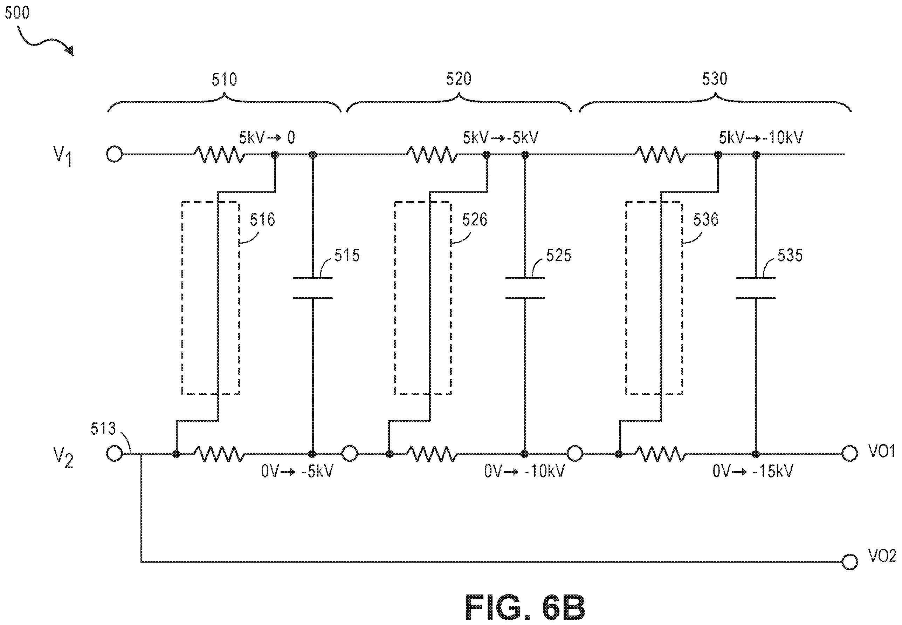

Pulse generator circuit 500 operates in a charge mode, and in a discharge mode. During the charge mode, described below with reference to FIG. 6A in more detail, capacitors 515, 525, and 535 are charged to a charge voltage by current received from the first and second power supply input terminals V1 and V2. During the discharge mode, described below with reference to FIG. 6B in more detail, capacitors 515, 525, and 535 are discharged to provide a current to a load (not shown) connected across first and second power output terminals VO1 and VO2.

FIG. 6A illustrates pulse generator circuit 500 during charge mode. First and second input voltages are respectively applied to first and second power supply input terminals V1 and V2 while each of switch stacks 516, 526, and 536 are nonconductive or open, and while first and second power output terminals may be disconnected from the load (not shown). Because each of switch stacks 516, 526, and 536 are open, substantially no current flows therethrough, and they are represented as open circuits in FIG. 6A. During the charge mode, each of capacitors 515, 525, and 535 are charged to a charge voltage by current flowing through resistors 512, 522, 532, 534, 524, and 514 to or toward a voltage equal to the difference between the first and second input voltages.

Each of the switches of switch stacks 516, 526, and 536 has a breakdown voltage rating which should not be exceeded. However, because the switches are serially connected, the capacitors 515, 525, and 535 may be charged to a voltage significantly greater than the breakdown voltage of the individual switches. For example, the breakdown voltage of the switches may be 1 kV, and the capacitors 515, 525, and 535 may be charged to a voltage of 5 kV, when 5 or more switches are used in each switch stack.

For example, the first and second input voltages may respectively be 5kV and 0V. In such an example, each of the capacitors 515, 525, and 535 is charged to or toward a voltage equal to 5kV. In some embodiments, the difference between the first and second input voltages is limited to be less than 10kV.

FIG. 6B illustrates pulse generator circuit 500 during discharge mode. First power supply input terminal V1 may be disconnected from the first input voltage. In some embodiments, first power supply input terminal V1 remains connected to the first input voltage. Second power supply input terminal V2 remains connected to the second input voltage. In addition, each of switch stacks 516, 526, and 536 are conductive or closed. Because each of switch stacks 516, 526, and 536 are closed, current flows therethrough, and they are represented as conductive wires in FIG. 6B. As a result, a low impedance electrical path from power supply input terminal V2 to power output terminal VO1 is formed by switch stack 516, capacitor 515, switch stack 526, capacitor 525, switch stack 536, and capacitor 535. Consequently, the difference between the voltages at the power output terminals VO1 and VO2 is equal to the number of stages (in this example, 3) times the difference between the first and second input voltages.

Where the first and second input voltages are respectively 5kV and 0V, a voltage difference of 15kV is developed across the power output terminals VO1 and VO2.

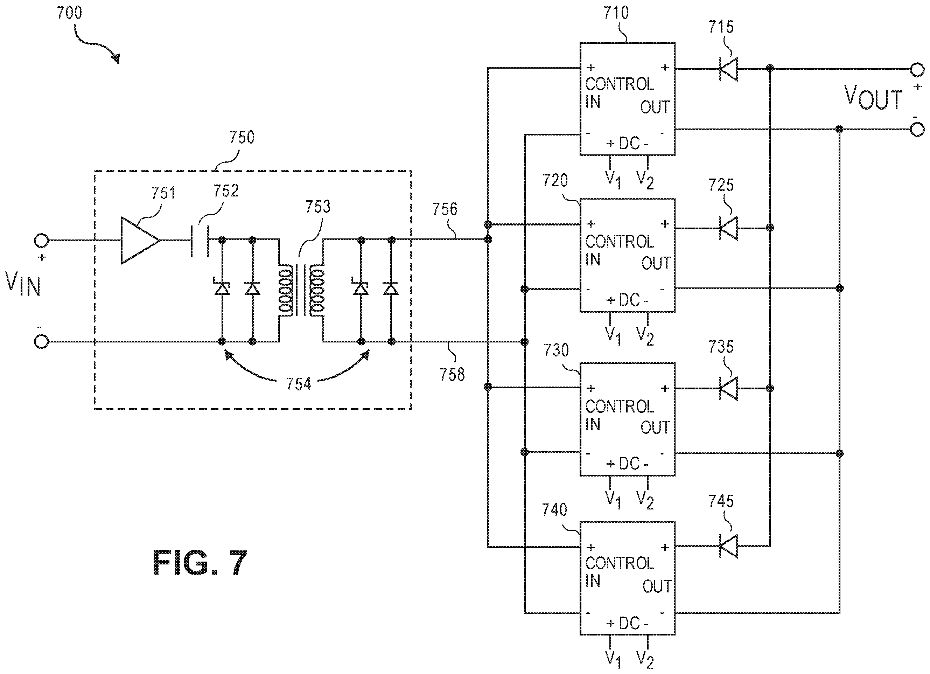

FIG. 7 illustrates an alternative pulse generator circuit 700 which may be used inside nsPEF system 100 of FIG. 1. This pulse generator includes panels in parallel. The number of panels can be adjusted to allow the system to generate different amounts of current and power.

Pulse generator circuit 700 receives input pulses across input port Vin, and generates output pulses across output port Vout in response to the received input pulses.

Pulse generator circuit 700 includes multiple panels or pulse generator circuits 710, 720, 730, and 740. Pulse generator circuit 700 also includes driver 750. In this embodiment, four pulse generator circuits are used. In alternative embodiments, fewer or more pulse generator circuits are used. For example, in some embodiments, 2, 3, 5, 6, 7, 8, 9, 10 or another number of pulse generator circuits are used.

Each of the pulse generator circuits 710, 720, 730, and 740 may have characteristics similar to other pulse generator circuits discussed herein. For example, each the pulse generator circuits 710, 720, 730, and 740 may have characteristics similar to pulse generator circuit 500 discussed above with reference to FIGs. 5, 6A, and 6B.

Each of pulse generator circuits 710, 720, 730, and 740 has positive and negative DC input terminals, positive and negative control input terminals, and positive and negative output terminals, and is configured to generate output voltage pulses across the positive and negative output terminals in response to driving signal pulses applied across the positive and negative control input terminals. The output voltage pulses are also based on power voltages received across positive and negative DC power input terminals.

The driving signal pulses are generated across conductors 756 and 758 by driver 750, which includes amplifier circuit 751, capacitor 752, and transformer 753. In some embodiments, driver 750 also includes clamp circuits 754.

Driver 750 receives an input signal pulse at input port Vin and generates a driving signal pulse across conductors 756 and 758 in response to the input signal pulse. Amplifier circuit 751 receives the input signal pulse and drives transformer 753 through capacitor 752, which blocks low frequency and DC signals. In response to being driven by amplifier circuit 751, transformer 753 generates an output voltage pulse across conductors 756 and 758, such that the duration of the output voltage pulse is equal to or substantially equal (e.g. within 10% or 1%) to the duration of the input signal pulse at input port Vin.

In some embodiments, clamp circuits 754 are included at least to dampen potential signals, which may otherwise be caused by resonance. Clamp circuits 754 include parallel diodes, which provide a short-circuit path for any current reversal, and also clamp the maximum voltage across the components connected to the clamp circuits 754.

In some embodiments, transformer 753 has a 1:1 turns ratio. In alternative embodiments, a different turns ratio is used.

Each of pulse generator circuits 710, 720, 730, and 740 receives the voltage pulses from driver 750 across the positive and negative control input terminals and generates corresponding voltage pulses across the positive and negative output terminals in response to the received voltage pulses from driver 750. The voltage pulses generated across the positive and negative output terminals have durations which are equal to or substantially equal (e.g. within 10% or 1%) to the durations of the voltage pulses received from driver 750.

In this embodiment, the negative output terminals of pulse generator circuits 710, 720, 730, and 740 are directly connected to the negative Vout terminal of the output port Vout of pulse generator circuit 700. In addition, in this embodiment, the positive output terminals of pulse generator circuits 710, 720, 730, and 740 are respectively connected to the positive Vout terminal of the output port Vout of pulse generator circuit 700 through diodes 715, 725, 735, and 745. Diodes 715, 725, 735, and 745 decouple pulse generator circuits 710, 720, 730, and 740 from one another. As a consequence, interference and the associated pulse distortion that would otherwise occur is substantially eliminated. For example, diodes 715, 725, 735, and 745 prevent current from one of pulse generator circuits 710, 720, 730, and 740 to another of pulse generator circuits 710, 720, 730, and 740 if the switching is not perfectly synchronous. Diodes 715, 725, 735, and 745 also prevent current from flowing from the pulse generator circuits 710, 720, 730, and 740 while they are charging.

In this embodiment, diodes 715, 725, 735, and 745 each include a single diode. In alternative embodiments, diodes 715, 725, 735, and 745 each include multiple diodes connected serially based at least upon voltage ratings of the serially connected diodes.

In this embodiment, diodes 715, 725, 735, and 745 are connected so as to conduct current from the positive terminal of output port Vout toward pulse generator circuits 710, 720, 730, and 740, as pulse generator circuits 710, 720, 730, and 740 in this embodiment are configured to generate negative pulses. In alternative embodiments, where pulse generator circuits are configured to generate positive pulses, diodes may be similarly connected so as to conduct current from the pulse generator circuits to the positive terminal of the output port.

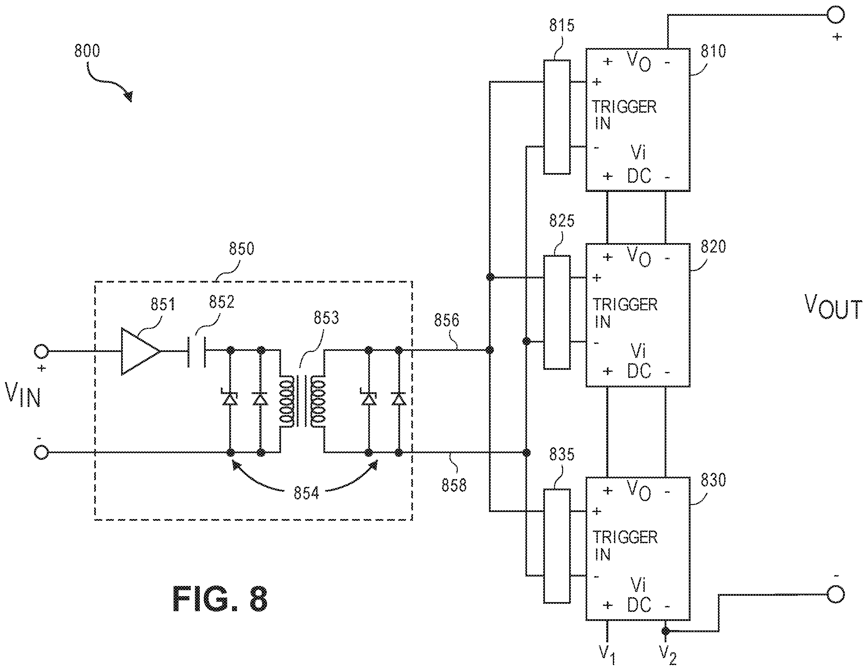

FIG. 8 illustrates a pulse generator circuit 800 which may be used for pulse generator circuits 710, 720, 730, and 740 of pulse generator circuit 1000 of FIG. 7.

Pulse generator circuit 800 receives input pulses across input port Vin, and generates output pulses across output port Vout in response to the received input pulses.

Pulse generator circuit 800 includes multiple pulse generator stages 810, 820, and 830. In this embodiment, pulse generator circuit 700 also includes driver 850, and optional common mode chokes 815, 825, and 835.

Each of the pulse generator stages 810, 820, and 830 may have characteristics similar to other pulse generator stages discussed herein. For example, each the pulse generator stages 810, 820, and 830 may have characteristics similar to stages 510, 520, and 530 of pulse generator circuit 500 discussed above with reference to FIGs. 5, 6A, and 6B. In some embodiments, fewer or more pulse generator stages may be used.

Each of pulse generator stages 810, 820, and 830 has positive and negative trigger input terminals, power positive and negative DC input terminals, and positive and negative Vo output terminals, and is configured to generate output voltage pulses across the positive and negative Vo output terminals in response to driving signal pulses applied across the positive and negative trigger input terminals. The output voltage pulses are also based on power voltages V1 and V2 respectively received at power positive and negative DC input terminals.

In this embodiment, the negative Vi input terminal of pulse generator stage 830 is connected with the negative terminal of the output port Vout of pulse generator circuit 800. In addition, in this embodiment, the negative Vo output terminal of pulse generator stage 810 is connected with the positive terminal of the output port Vout of pulse generator circuit 800.

In addition, as shown, the positive Vo output terminal of pulse generator stage 830 is connected with the positive Vi input terminal of pulse generator stage 820, and the negative Vo output terminal of pulse generator stage 830 is connected with the negative Vi input terminal of pulse generator stage 820. Furthermore, the positive Vo output terminal of pulse generator stage 820 is connected with the positive Vi input terminal of pulse generator stage 810, and the negative Vo output terminal of pulse generator stage 820 is connected with the negative Vi input terminal of pulse generator stage 810.

The driving signal pulses for pulse generator stages 810, 820, and 830 are generated across conductors 856 and 858 by driver 850, which includes amplifier circuit 851, capacitor 852, and transformer 853. In some embodiments, driver 850 also includes clamp circuits 854.

Driver 850 receives an input signal pulse at input port Vin, which is connected to conductors 756 and 758, as shown in FIG. 7 discussed above. Driver 850 generates a driving signal pulse across conductors 856 and 858 in response to the input signal pulse. Amplifier circuit 851 receives the input signal pulse, and drives transformer 853 through capacitor 852, which reduces or blocks low frequency and DC signals. In response to being driven by amplifier circuit 851, transformer 853 generates an output voltage pulse across conductors 756 and 758, such that the duration of the output voltage pulse is equal to or substantially equal (e.g. within 10% or 1%) to the duration of the input signal pulse at input port Vin.

In some embodiments, clamp circuits 854 are included at least to dampen potential signals, which may otherwise be caused by resonance. Clamp circuits 854 include parallel diodes, which provide a short-circuit path for any current reversal, and also clamp the maximum voltage across the components connected to the clamp circuits 854.

In some embodiments, transformer 853 has a 1:1 turns ratio. In alternative embodiments, a different turns ratio is used.

Each of pulse generator stages 810, 820, and 830 receives the voltage pulses from driver 850 through a corresponding choke 815, 825, or 835, which blocks high frequency signals, for example, from coupling from the high voltage pulse generator stages 810, 820, and 830. The voltage pulses are received at the positive and negative trigger input terminals and the pulse generator stages 810, 820, and 830 each generate corresponding voltage pulses across the positive and negative Vo output terminals in response to the received voltage pulses from driver 850. The voltage pulses generated across the positive and negative Vo output terminals have durations which are equal to or substantially equal (e.g. within 10% or 1%) to the durations of the voltage pulses received from driver 850.

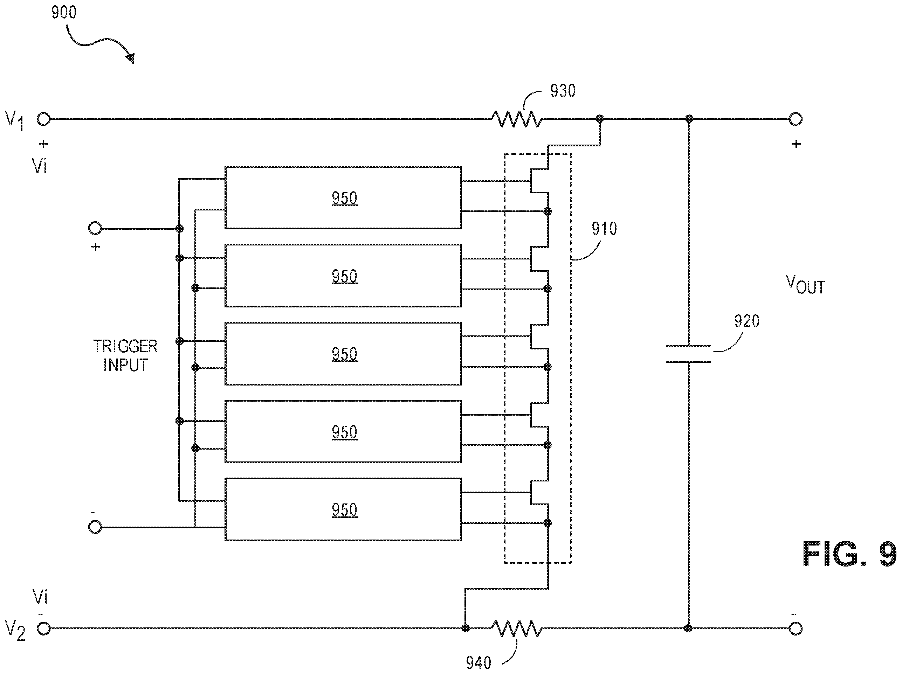

FIG. 9 illustrates a pulse generator stage 900 which may be used as one of the pulse generator stages 810, 820, and 830 of pulse generator circuit 800 shown in FIG. 8.

Pulse generator stage 900 receives trigger pulses across input port trigger input, and generates output voltages at output port Vout in response to the received trigger pulses. The output voltages are also generated based on power voltages received at power input terminals V1 and V2. Pulse generator stage 900 includes multiple switch drivers 950. Pulse generator stage 900 also includes switch stack 910, capacitor 920, and resistors 930 and 940.

Switch drivers 950 are configured to receive the trigger pulses, and to generate control signals for the switches of switch stack 910 in response to the received trigger pulses, as discussed in further detail below. Each of the control signals is referenced to a voltage specific to the switch being driven. Accordingly, a first switch receives a control signal pulse between first and second voltages, and a second switch receives a control signal pulse between third and fourth voltages, where each of the first, second, third, and fourth voltages are different. In some embodiments, the difference between the first and second voltages is substantially the same as the difference between the third and fourth voltages.

Switch stack 910, capacitor 920, and resistors 930 and 940 cooperatively function with corresponding elements in the other pulse generator stages of pulse generator circuit 800, discussed above with reference to FIG. 8, to generate the voltage pulses across the positive and negative Vo output terminals of pulse generator circuit 800. These elements may, for example, cooperatively function as the corresponding elements discussed above with reference to pulse generator circuit 500 shown in FIGs. 5, 6A, and 6B. For example, these elements may cooperate to generate the voltage pulses across the positive and negative Vo output terminals of pulse generator circuit 800 in response to the power voltages applied to power input terminals V1 and V2 and to the control signals applied to the switches of switch stack 910.

Because the control signals are generated in response to the input pulses received across input port Vin of pulse generator circuit 700 illustrated in FIG. 7 through multiple stages of driving, the control signals cause all of the switches of the switch stacks of pulse generator circuit 700 to be turned on and to be turned off substantially simultaneously. For example, a 15V input pulse having a duration of, for example 100 ns, received at input port Vin of pulse generator circuit 700 may cause the pulse generator circuit 700 to generate a high-voltage (e.g. ~15 kV) output pulse having a duration of about 100 ns. Similarly, a 15V input pulse having a duration of, for example 5 µs, received at input port Vin of pulse generator circuit 700 may cause the pulse generator circuit 700 to generate a high-voltage (e.g. ~15 kV) output pulse having a duration of about 5 µs. Accordingly, the duration of the high-voltage output pulse is substantially the same as a selected duration of an input pulse.

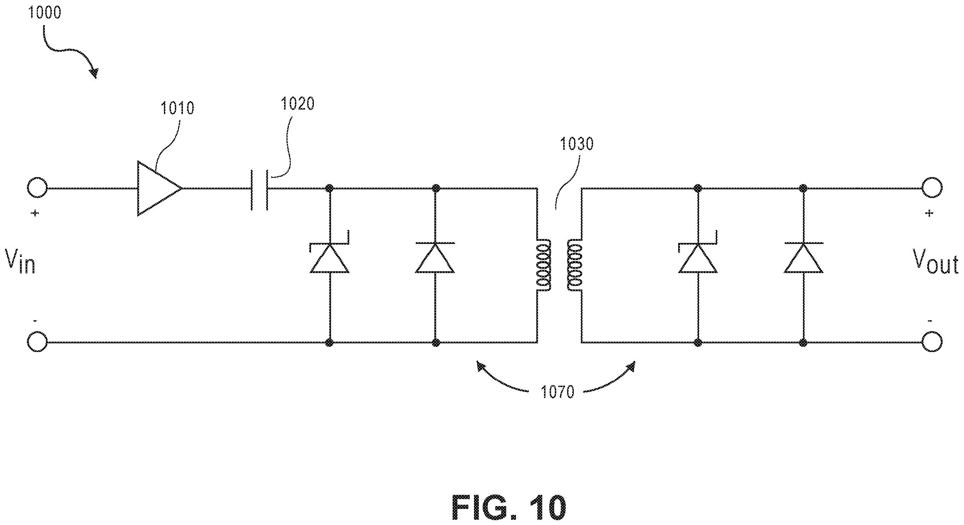

FIG. 10 illustrates a switch driver 1000 which may be used as one of the switch drivers shown in FIG. 9.

Switch driver 1000 receives trigger pulses across input port Vin, and generates control signal pulses at output port Vout in response to the received trigger pulses. Switch driver 1000 includes amplifier circuit 1010, capacitor 1020, and transformer 1030. In some embodiments, switch driver 1000 also includes clamps circuits 1070.

Amplifier circuit 1010 receives the trigger pulses, and drives transformer 1030 through capacitor 1020, which reduces or blocks low frequency and DC signals. In response to being driven by amplifier circuit 1010, transformer 1030 generates control signal pulses at output port Vout, such that the duration of the control signal pulses is equal to or substantially equal (e.g. within 10% or 1%) to the duration of the trigger pulses at input port Vin.

In some embodiments, amplifier circuit 1010 includes multiple amplifier integrated circuits. For example, for increased current driving capability, multiple amplifier integrated circuits may be connected in parallel to form amplifier circuit 1010. For example, 2, 3, 4, 5, 6, 7, 8 or another number of amplifier integrated circuits may be used.

In some embodiments, clamp circuits 1070 are included at least to dampen potential signals, which may otherwise be caused by resonance. Clamp circuits 1070 include parallel diodes, which provide a short-circuit path for any current reversal, and also clamp the maximum voltage across the components connected to the clamp circuits 1070.

In some embodiments, the drivers 750, 850, and 1000 receive power from a DC-DC power module which is isolated from the power supply for the Marx generator. This ensures the cutoff of ground coupling.

In some embodiments, transformer 1030 has a 1:1 turns ratio. In alternative embodiments, a different turns ratio is used.

In some embodiments, in order to obtain very fast switching, the transformers 1030 has fewer than 5 turns in the primary winding and fewer than 5 turns in the secondary winding. For example, in some embodiments, the transformer 1030 has 1, 2, 3, or 4 turns in each of the primary and secondary windings. In some embodiments, the transformer 1030 has less than a complete turn, for example, ½ turn in the primary and secondary windings. The low number of turns in each of the primary and secondary windings allows for a low inductance loop and increases the current risetime in the secondary winding, which charges the input capacitance of the MOSFET switches.

Transformers for triggering MOSFETs in conventional applications require high coupling, high permeability, and a low-loss core in order to ensure current transfer efficiency. From pulse to pulse, the residual flux in the core needs to be cleared in order to avoid saturation when the transformer is operated at high frequency. Conventionally, a resetting circuit, which involves a third winding, to dissipate the core energy is used.

In some embodiments, lossy transformers, such as that typically used as an electromagnetic interference (EMI) choke to confine high frequency signals and dissipate their energy as heat are used to trigger the switches. For example, the transformers may have a voltage time constant less than 100Vµs. In some embodiments, the Transformers have a voltage time constant less than 50Vµs, 30Vµs, 20Vµs, 10Vµs, or 5Vµs. The use of the lossy transformer is contrary to the common practice in power electronics.

Although the high frequency flux is dampened due to the loss of the core (eddy loss, hysteresis loss, and resistive loss), the lossy transformers still allow sufficient confinement of the magnetic flux and provides sufficient coupling. In addition, the flux also decreases quickly in response to the signal on the primary winding being removed. The flux decay process usually takes approximately several microseconds.

Having such a transformer conventionally seems disadvantageous, but for coupling nanosecond to a few microsecond pulses, such a transformer is preferably used. Consequently, the following benefits are achieved: 1) high voltage, high frequency transient coupling from the high-voltage Marx generators to the low-voltage drivers is suppressed; 2) because of the loss in the transformer cores, the residual flux from previous pulses are dissipated faster than common low-loss transformer cores, such that the resetting winding is not needed and is not present.

A benefit of the switch driver 1000 is that it limits the output pulse duration. Because the switch control signals are generated by transformer 1030, even if circuitry generating the input trigger signals at input port Vin were to generate a pulse of indefinite length, the transformer would saturate, causing the control signals to turn off the switches.

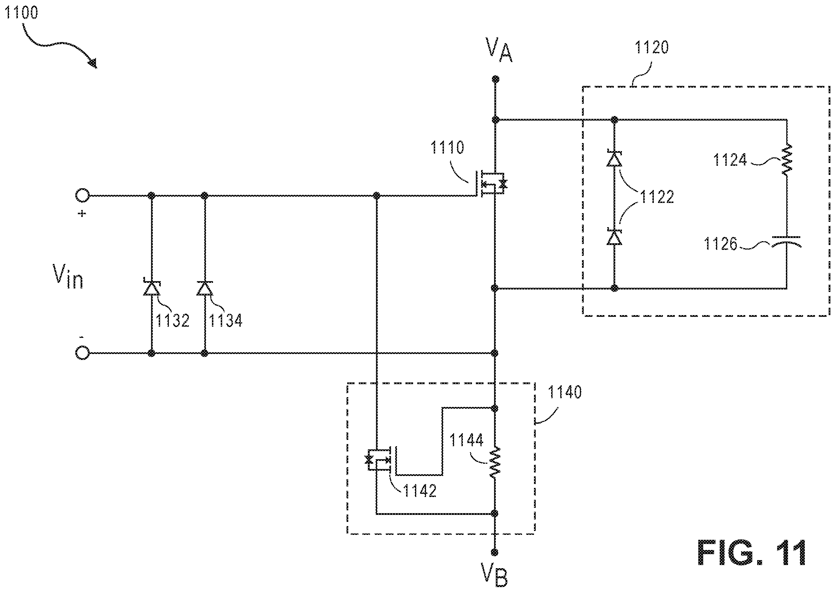

FIG. 11 illustrates an example of a switch element 1100 comprising components which may be used in the switch stacks discussed here. Switch element 1100 includes switch 1110, and selectively forms a conductive or low resistance path between terminals VA and VB in response to a control voltage applied to input port Vin.

In some embodiments, switch 1110 is a transistor, such as a MOSFET. In some embodiments, switch 1110 is another type of switch. In some embodiments, switch 1110 has a turn on time of less than 5 ns, about 5 ns, about 10 ns, about 25 ns, about 15 ns, about 75 ns, about 100 ns, or greater than 100 ns.

In some embodiments, switch element 1100 also includes snubber circuit 1120. In some embodiments, the turn on times of the switches of the switch stacks are not identical. In order to prevent voltages greater than that which switch 1110 can tolerate, snubber circuit 1120 provides a current shunt path bypassing switch 1110. Diodes 1122 provide a low-frequency current path, and the combination of the capacitor 1126 and resistor 1124 provide a high-frequency current path.

In some embodiments, switch element 1100 also includes optional overcurrent protection circuit 1140. Overcurrent protection circuit 1140 includes switch 1142 and sense resistor 1144.

Current flowing from terminal VA to terminal VB is conducted through sense resistor 1144. Accordingly, a voltage is generated across sense resistor 1144 when the current flows from terminal VA to terminal VB. The generated voltage controls a conductive state of switch 1142. If the current flowing from terminal VA to terminal VB is greater than a threshold, the generated voltage causes the switch 1142 to conduct. As a result, switch 1142 reduces the control voltage of switch 1110. In response to the reduced control voltage, switch 1110 becomes less conductive or turns off. Consequently, the current which may be conducted from terminal VA to terminal VB is limited by overcurrent protection circuit 1140.

In some embodiments, a current limiting resistor is placed between the gate of switch 1110 and the drain of switch 1142 to prevent switch 1142 from experiencing current greater than that which would cause damage.

In the embodiments discussed herein, MOSFET switches are used. In alternative embodiments, other switches are used. For example, in some embodiments, thyristors, IGBTs or other semiconductor switches are used.

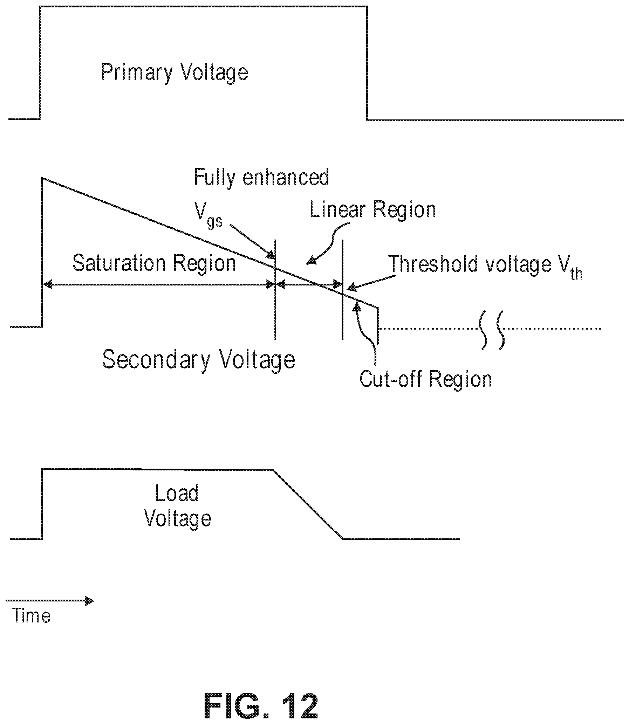

An example of the operation of the transformer is illustrated in FIG. 12. The voltage at the input primary inductor is substantially a square waveform, but the voltage at the secondary inductor, which is the MOSFET's gate-source voltage, tapers as the voltage magnitude decreases toward zero, for example, within a period of several microseconds. After a reduction in voltage at the secondary inductor due to transformer saturation, the switch receiving the voltage enters a linear region of operation from a saturation region of operation when the voltage is less than the fully enhanced Vgs. As a result, the resistance of the switch increases and the output voltage across the load also shows a tapered profile. When the voltage at the secondary inductor decreases to a value less than the turn-on threshold of a MOSFET (Vth), the MOSFET will be shut off. Once the MOSFET is off, even if the duration of the trigger signal is extended, the switch no longer conducts and can be considered an open circuit. The waveform of the voltage at the secondary inductor therefore limits the duration of high voltage output pulses from each panel, for example, to be several microseconds or less.

In some embodiments, the duration of the trigger signal is short enough that the switches remain in saturation because the reduction in voltage at the secondary inductor is insufficient to cause the switches to enter linear region operation. In such embodiments, the load voltage pulses do not exhibit the tapering illustrated in FIG. 12. For example, in such embodiments the load voltage pulses may be substantially square.

In some embodiments, the switch stacks discussed herein include switches, as discussed above, as well as other components.

In some embodiments, when generating pulses of a duration less than a threshold, the shape of the pulses are substantially square. In some embodiments, when generating pulses of the duration greater than a threshold, the shape of the pulses are substantially square for a duration substantially equal (e.g. within 10% or 1%) to the threshold. During the time after the threshold, the voltage of such long pulses drops toward 0 V. In some embodiments, the drop toward 0 V is substantially linear. In some embodiments, the drop toward 0 V is substantially exponential.

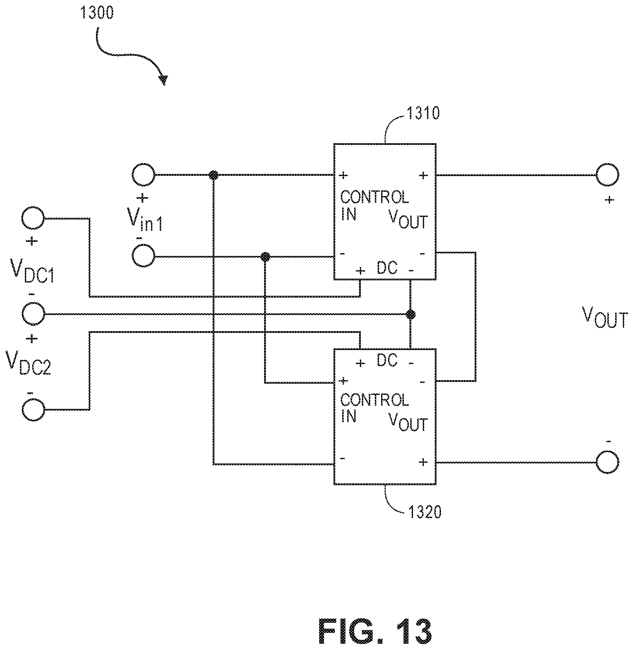

FIG. 13 illustrates an alternative pulse generator circuit 1300 which may be used inside nsPEF system 100 of FIG. 1.

Pulse generator circuit 1300 receives input pulses across input port Vin and DC voltages at input ports VDC1 and VDC2, and generates output pulses across output port Vout in response to the received input pulses and DC voltages.

Pulse generator circuit 1300 includes multiple pulse generator circuits 1310 and 1320. In this embodiment, two pulse generator circuits are used. In alternative embodiments, more pulse generator circuits are used. For example, in some embodiments, 3, 4, 5, 10 or another number of pulse generator circuits having their output ports serially connected, as discussed below with reference to pulse generator circuit 1300, are used.

Each of pulse generator circuits 1310 and 1320 may be similar to the other pulse generator circuits discussed herein. For example pulse generator circuits 1310 and 1320 may be similar to or may be substantially identical to pulse generator circuit 700 discussed above with reference to FIG. 7.

Each of pulse generator circuits 1310 and 1320 receive the same input pulse signal across their respective Control In input ports. In response, each of pulse generator circuits 1310 and 1320 generate high voltage pulses across their respective Vout output ports. Because the Vout output ports of pulse generator circuits 1310 1320 are serially connected, the voltage pulse generated by pulse generator circuits 1310 and 1320 across output port Vout of pulse generator circuit 1300 is substantially equal (e.g. within 10% or 1%) to the sum of the voltages of the pulses respectively generated by pulse generator circuits 1310 and 1320.

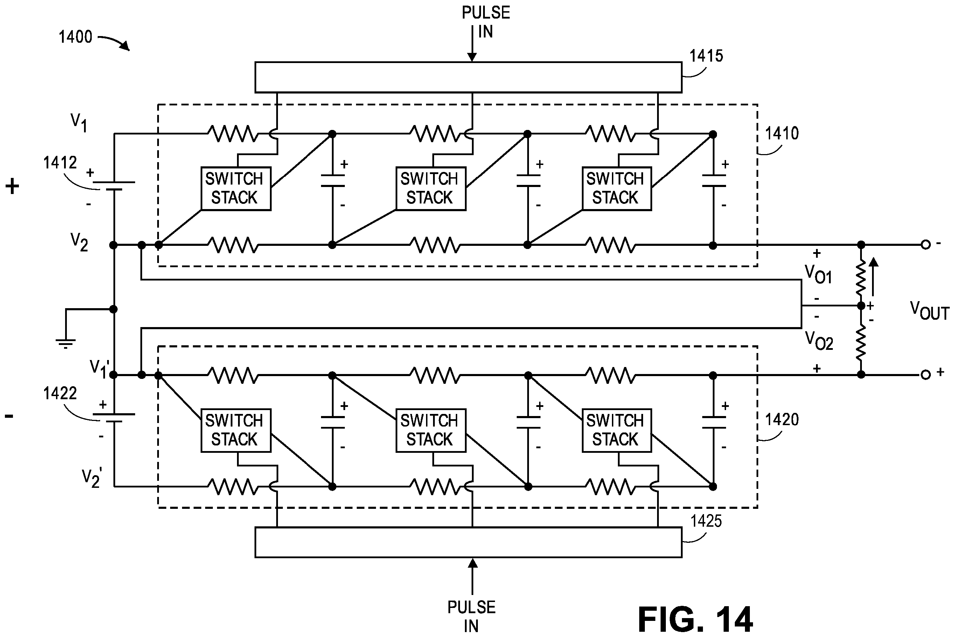

FIG. 14 illustrates an alternative pulse generator circuit 1400 which may be used inside nsPEF system 100 of FIG. 1, and which has characteristics similar to the pulse generator circuit 1300 of FIG. 13. Pulse generator circuit 1400 includes pulse generators 1410 and 1420, drivers 1415 and 1425, and power supplies 1412 and 1422.

Pulse generator circuit 1400 includes multiple pulse generator circuits 1410 and 1420. In this embodiment, two pulse generator circuits are used. In alternative embodiments, more pulse generator circuits are used. Each of pulse generator circuits 1410 and 1420 may be similar to the other pulse generator circuits discussed herein.

Pulse generator circuit 1400 receives input pulses at each of drivers 1415 and 1425, which may be similar to driver 850 discussed above with reference to FIG. 8. Pulse generator circuit 1400 generates output pulses across output port Vout in response to the received input pulses. The output voltage pulses are also based on power voltages received from power supplies 1412 and 1422.

Each of drivers 1415 and 1425 receive an input pulse signal. In response to the received input signals, drivers 1415 and 1425 respectively generate driving signal pulses for pulse generator circuits 1410 and 1420. In response to the driving signal pulses, each of pulse generator circuits 1410 and 1420 generate high voltage pulses across their respective output ports Vo1 and Vo2. Because the Vo1 and Vo2 output ports of pulse generator circuits 1410 and 1420 are serially connected, the voltage pulse generated by pulse generator circuits 1410 and 1420 across output port Vout of pulse generator circuit 1400 is substantially equal (e.g. within 10% or 1%) to the sum of the voltages of the pulses respectively generated by pulse generator circuits 1410 and 1420.

In this embodiment, pulse generator circuit 1410 generates a high voltage pulse across its output port Vo1 which is substantially equal (e.g. within 10% or 1%) to three times the voltage of power supply 1412, (-3 x [V1 - V2]). In addition, pulse generator circuit 1420 generates a high voltage pulse across its output port Vo2 which is substantially equal (e.g. within 10% or 1%) to three times the voltage of power supply 1422 (3 x [V' 1 - V'2]). As a result, pulse generator circuit 1400 generates a voltage of (3 x [V' 1 - V'2]) - (-3 x [V1 - V2]) across its output port Vout.

In some embodiments, a single driver circuit connected to both pulse generator circuit 1410 and 1420 is used instead of drivers 1415 and 1425. In such embodiments, the single driver circuit generates driving signal pulses for both pulse generator circuits 1410 and 1420 in response to an input pulse signal.

For various purposes, it may be desirable to discharge the capacitors which are used by a pulse generator to generate pulses. For example, it may be desirable to discharge capacitor 920 of the pulse generator stage 900 of FIG. 9. Discharging the capacitors may be done using various embodiments of various discharge circuits. Some embodiments are discussed herein.

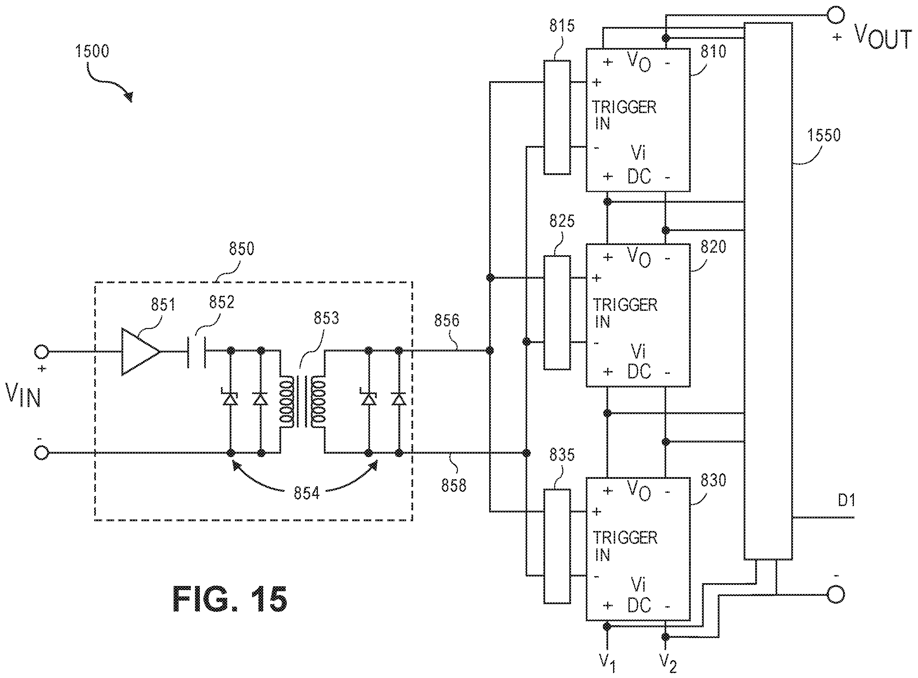

FIG. 15 illustrates a pulse generator circuit 1500 which may be used for pulse generator circuits 710, 720, 730, and 740 of pulse generator circuit 700 of FIG. 7. Pulse generator circuit 1500 is similar to pulse generator circuit 800 illustrated in FIG. 8. Pulse generator circuit 1500 additionally includes a particular discharge circuit 1550.

As shown, discharge circuit 1550 is electrically connected to first and second power supply input terminals V1 and V2. Discharge circuit 1550 is also electrically connected to discharge input terminal D1. Based on voltages at the first and second power supply input terminals V1 and V2 and discharge input terminal D1, discharge circuit 1550 selectively discharges each of the pulse generator stages 810, 820, and 830.

In some embodiments, discharge circuit 1550 is configured to discharge each of the pulse generator stages 810, 820, and 830 in response to a discharge control signal received at discharge input terminal D1. In some embodiments, discharge circuit 1550 is configured to respectively discharge each of the pulse generator stages 810, 820, and 830 in response to a comparison of the charging voltage as determined by the voltages at first and second power supply input terminals V1 and V2 and the charged voltage stored on the capacitor of each of the pulse generator stages 810, 820, and 830.

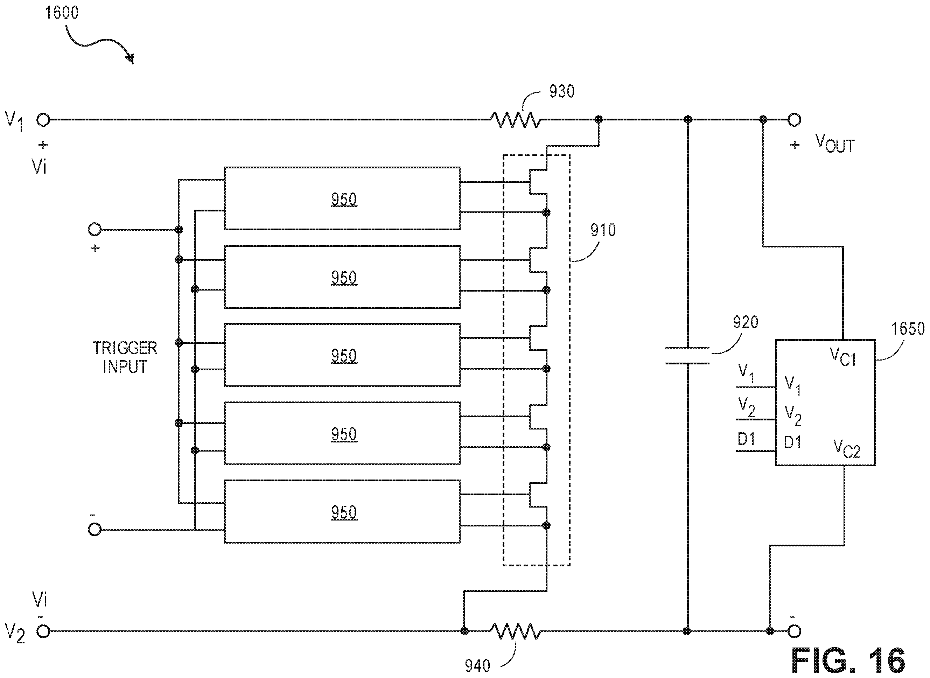

FIG. 16 illustrates a pulse generator stage 1600 which may be used as one of the pulse generator stages 810, 820, and 830 of pulse generator circuit 1500 shown in FIG. 15. Pulse generator stage 1600 includes a discharge circuit stage 1650.

In some embodiments, discharge circuit stage 1650 is configured to discharge capacitor 920 in response to a discharge command signal received at discharge input terminal D1. In some embodiments, discharge circuit stage 1650 is configured to discharge capacitor 920 in response to a comparison of the charging voltage as determined by the voltages at first and second power supply input terminals V1 and V2 and the charged voltage stored on the capacitor 920.

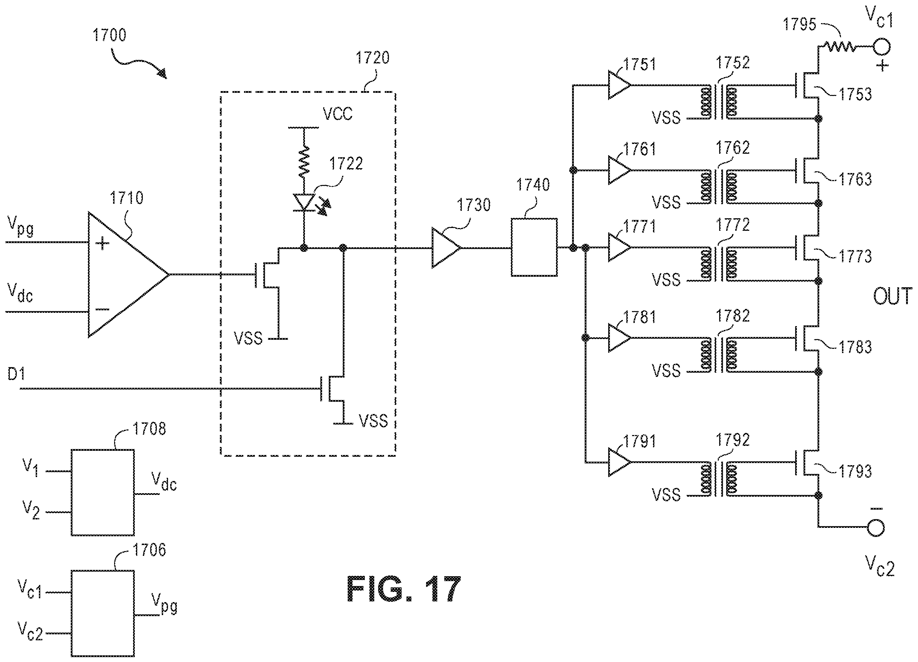

FIG. 17 is a schematic illustration of an embodiment of a discharge circuit stage 1700 used in the pulse generator stage 1600 of FIG. 16. Pulse generator stage 1700 includes voltage generators 1706 and 1708, comparator 1710, OR circuit 1720, buffer 1730, pulse generator 1740, buffers 1751, 1761, 1771, 1781, and 1791, transformers 1752, 1762, 1772, 1782, and 1792, switches 1753, 1763, 1773, 1783, and 1793, and resistor 1795.

Comparator 1710 is configured to generate a signal which selectively causes the other components of discharge circuit stage 1700 to cause switches 1753, 1763, 1773, 1783, and 1793, to become conductive and to effectively short out the discharge terminals at output port OUT. Using multiple switches has the benefit of allowing for voltages at the output of discharge circuit stage 1700 to be greater than the maximum drain/source voltage rating of a single switch. For example, in the illustrated embodiment, five switches are used. If the maximum drain/source voltage rating for each switch is 1000 V, using five switches ideally allows for 5000 V at the output of discharge circuit stage 1700.

For example, in this embodiment, comparator 1710 receives input voltages at voltage control input terminals Vpg and Vdc. The voltage at voltage input terminal Vpg is generated by voltage generator 1706 based on the voltage across the capacitor to be selectively discharged by discharge circuit stage 1700. The voltage at voltage input terminal the Vdc is generated by voltage generator 1708 based on the voltages of the first and second power supply input terminals V1 and V2.

In some embodiments, voltage generators 1706 and 1708 are level shift circuits which receive voltages which are higher than that which comparator 1710 is able to withstand. For example, voltage generator 1706 may be configured to receive a voltage difference across its inputs Vc1 and Vc2 of about 5 kV, and to generate an output voltage on terminal Vpg equal to about 10 V, where the output voltage on terminal Vpg is proportional to the voltage difference across inputs Vc1 and Vc2. Similarly, voltage generator 1708 may be configured to receive a voltage difference across its inputs V1 and V2 of about 5 kV, and to generate an output voltage on terminal Vdc equal to about 10 V, where the output voltage on terminal Vdc is proportional to the voltage difference across inputs V1 and V2.

In some embodiments, the proportionality constant relating the voltage on terminal Vpg to the voltages across inputs Vc1 and Vc2 is equal to the proportionality constant relating the voltage on terminal Vdc to the voltages across inputs V1 and V2. In such embodiments, comparator 1710 is configured to cause switches 1753, 1763, 1773, 1783, and 1793, to become conductive and provide a discharge path across the output port OUT in response to the DC input voltage across terminals V1 and V2 of pulse generator stage 1600 being less than the voltage across capacitor 920 of pulse generator stage 1600.

In some embodiments, the proportionality constant relating the voltage on terminal Vpg to the voltages across inputs Vc1 and Vc2 is not equal to the proportionality constant relating the voltage on terminal Vdc to the voltages across inputs V1 and V2. In such embodiments, comparator 1710 is configured to cause switches 1753, 1763, 1773, 1783, and 1793, to become conductive and to effectively short out the output port OUT in response to the DC input voltage across terminals V1 and V2 of pulse generator stage 1600 being less than the voltage across capacitor 920 of pulse generator stage 1600 by more than a predetermined threshold related to the difference in the proportionality constants.

For example, in some embodiments, a voltage difference of 5 kV across input terminals V1 and V2 causes voltage generator 1708 to generate a voltage of 10 V at terminal Vdc, and a voltage difference of 5.1 kV across input terminals Vc1 and Vc2 causes voltage generator 1706 to generate a voltage of 10 V at terminal Vpg. In such embodiments, comparator 1710 is configured to cause switches 1753, 1763, 1773, 1783, and 1793 to become conductive and to effectively short out the output port OUT in response to the DC input voltage across terminals V1 and V2 of pulse generator stage 1600 being more than 100 V less than the voltage across capacitor 920 of pulse generator stage 1600.

In some embodiments, voltage generators 1706 and 1708 are resistive voltage dividers, each comprising first and second resistive elements serially connected. The output voltage is generated at the node shared by the first and second resistive elements, and first and second input voltages are respectively connected with one of the first and second resistive elements.

OR circuit 1720 is configured to selectively generate a signal which causes switches 1753, 1763, 1773, 1783, and 1793 to become conductive and to effectively short out the output port OUT in response to the DC input voltage across terminals V1 and V2 of pulse generator stage 1600. OR circuit 1720 is configured to generate the signal based on the output of comparator 1710 and on the voltage level applied at discharge control input terminal D1.

In this embodiment, OR circuit 1720 is configured to causes switches 1753, 1763, 1773, 1783, and 1793 to become conductive in response to either the output of comparator 1710 or the voltage level at discharge input terminal D1 being greater than a threshold. For example, if either the output of comparator 1710 or the voltage level at discharge input terminal D1 is greater than the threshold, if either the output of comparator 1710 or the voltage level at discharge input terminal D1 is greater than the threshold, the output of OR circuit 1720 causes switches 1753, 1763, 1773, 1783, and 1793 to become conductive.

In this embodiment, OR circuit 1720 includes light emitting diode (LED) 1722, which is configured to emit light when either the output of comparator 1710 or the voltage level at discharge input terminal D1 is greater than the threshold. Accordingly, LED 1722 provides a visual indication that the discharge circuit stage 1700 is discharging the capacitor 920 of pulse generator stage 1600.

Discharge circuit stage 1700 optionally includes buffer 1730. The buffer 1730 receives the signal generated by OR circuit 1720, and generates an output signal for pulse generator 1740.

In some embodiments, buffer 1730 is not used. In such embodiments, the signal generated by OR circuit 1720 may be provided directly to pulse signal generator 1740, or may be conditioned by other circuitry, which provides a signal to pulse signal generator 1740 based on the signal generated by OR circuit 1720.

In some embodiments, buffer 1730 is an inverting buffer. In some embodiments, buffer 1730 is a non-inverting buffer.

In this embodiment, pulse signal generator 1740 is configured to receive the signal from buffer 1730. In response to the received signal, pulse signal generator 1740 selectively generates a series of pulse signals based on the received signal. In some embodiments, the received signal is received by an enable input, such that pulse signal generator 1740 generates the series of pulse signals in response to the received signal being of an appropriate logic state, and does not generate the series of pulse signals in response to the received signal being of an opposite logic state.

In some embodiments, pulse signal generator 1740 includes a timer circuit, such as a 555 timer. In such embodiments, the timer circuit may be configured to generate pulse signals appropriate for causing switches 1753, 1763, 1773, 1783, and 1793 to become conductive. For example, timer circuit may be tuned so as to generate a series of pulse signals which are appropriate for transformers 1752, 1762, 1772, 1782, and 1792, such that transformers 1752, 1762, 1772, 1782, and 1792 do not saturate and such that transformers 1752, 1762, 1772, 1782, and 1792 generate signals which cause switches 1753, 1763, 1773, 1783, and 1793 to be conductive for a large portion of each period of the pulse signal series. For example, frequency, duty cycle, rise time, and fall time may be tuned to avoid saturation of the transformers 1752, 1762, 1772, 1782, and 1792, and may be tuned to increase or maximize the portion of each period during which switches 1753, 1763, 1773, 1783, and 1793 are conductive.

Discharge circuit stage 1700 optionally includes buffers 1751, 1761, 1771, 1781, and 1791. The buffers 1751, 1761, 1771, 1781, and 1791 receive the series of pulse signals generated by pulse signal generator 1740, and respectively generate signals for transformers 1752, 1762, 1772, 1782, and 1792.

In some embodiments, buffers 1751, 1761, 1771, 1781, and 1791 are not used. In such embodiments, the signal generated by pulse signal generator 1740 may be provided directly to the transformers 1752, 1762, 1772, 1782, and 1792, or may be conditioned by other circuitry, which provides a signal to the transformers 1752, 1762, 1772, 1782, and 1792 based on the signal generated by pulse signal generator 1740.

In some embodiments, buffers 1751, 1761, 1771, 1781, and 1791 are inverting buffers. In some embodiments, buffers 1751, 1761, 1771, 1781, and 1791 are non-inverting buffers.

In this embodiment, transformers 1752, 1762, 1772, 1782, and 1792 are configured to receive the pulse signal series from buffers 1751, 1761, 1771, 1781, and 1791. In response to the received pulse signal series, transformers 1752, 1762, 1772, 1782, and 1792 selectively generates a series of pulses based on the received pulse signal series. The series of pulse signals generated by transformers 1752, 1762, 1772, 1782, and 1792 respectively cause switches 1753, 1763, 1773, 1783, and 1793 to become conductive and to effectively short out the output port OUT.

For example, switches 1753, 1763, 1773, 1783, and 1793 may be transistors, and in response to the received pulse signal series, each of transformers 1752, 1762, 1772, 1782, and 1792 may be configured to generate a gate voltage and a source voltage for a corresponding one of the transistors 1753, 1763, 1773, 1783, and 1793. Because of the floating output of the transformers 1752, 1762, 1772, 1782, and 1792, the gate voltages are generated so as to be referenced to the corresponding source voltages. In some embodiments, bipolar transistors may be used and the transformers 1752, 1762, 1772, 1782, and 1792 may be configured to generate a base voltage and an emitter voltage for a corresponding one of the transistors 1753, 1763, 1773, 1783, and 1793.

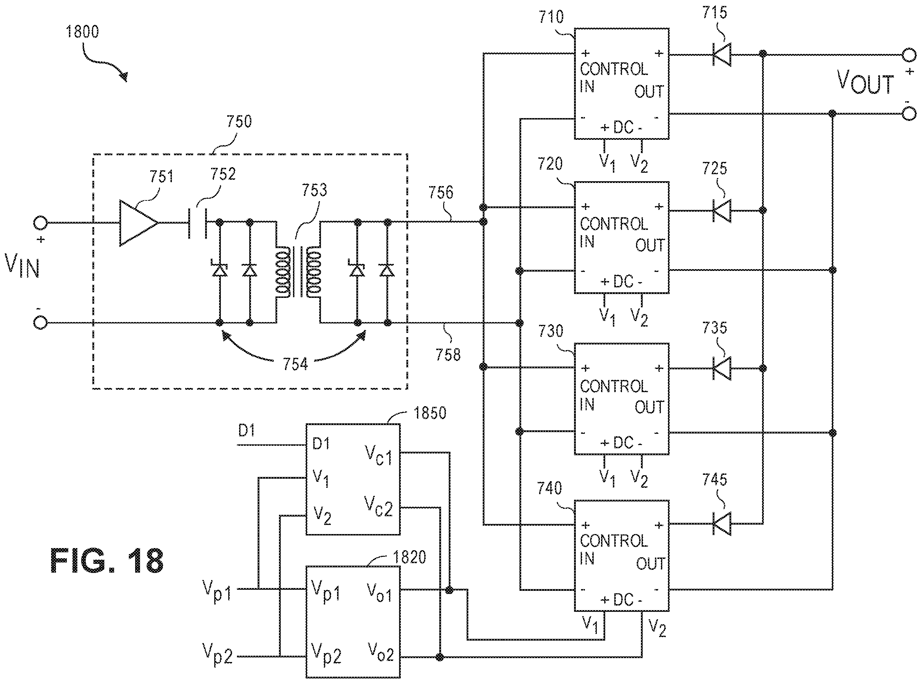

FIG. 18 illustrates an alternative pulse generator circuit 1800 which may be used for pulse generator circuit 700 of FIG. 7. Pulse generator circuit 1800 is similar to pulse generator circuit 700 illustrated in FIG. 7. Pulse generator circuit 1800 additionally includes a discharge circuit 1850 and a peak voltage source 1820.

As shown, discharge circuit 1850 is electrically connected to first and second power supply input terminals VP1 and VP2. Discharge circuit 1850 is also electrically connected to discharge input terminal D1. Based on voltages at the first and second power supply input terminals VP1 and VP2, discharge input terminal D1, and first and second power supply terminals V1 and V2, discharge circuit 1850 selectively discharges first and second power supply terminals V1 and V2.

In some embodiments, discharge circuit 1850 is configured to discharge first and second power supply terminals V1 and V2 in response to a discharge control signal received at discharge input terminal D1. In some embodiments, discharge circuit 1850 is configured to discharge the first and second power supply terminals V1 and V2 in response to a comparison of the charging voltage as determined by the voltages at first and second power supply terminals V1 and V2 and the voltage of the first and second power supply input terminals VP1 and VP2.

Discharge circuit stage 1700 of FIG. 17, or any of the other discharge circuits discussed herein may be used as discharge circuit 1850.

Peak voltage source 1820 may be any low-pass filter. For example, peak voltage source 1820 may include a resistor and a capacitor to form a single pole RC filter. Other filters may additionally or alternatively be used.

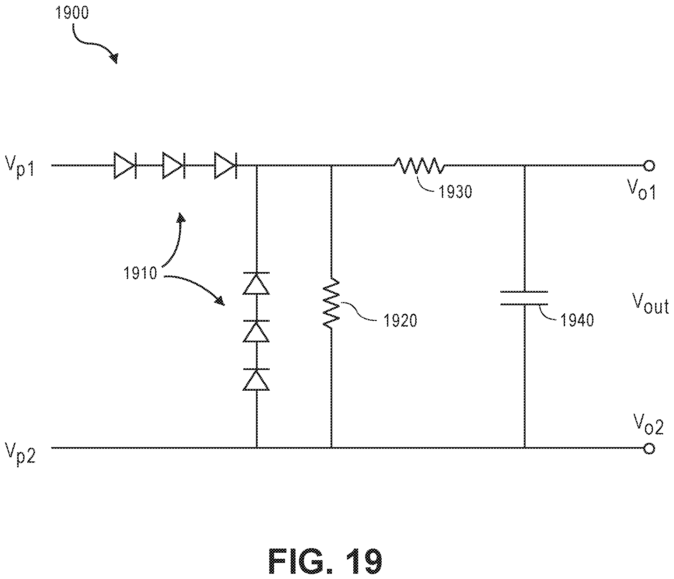

FIG. 19 is an embodiment of a peak voltage source 1900, which may be used as peak voltage source 1820 of FIG. 18. As shown, peak voltage source 1900 includes diodes 1910, resistor 1920, RC resistor 1930, and RC capacitor 1940. Resistor 1920 operates to passively discharge capacitor 1940 and power supply terminals V1 and V2.

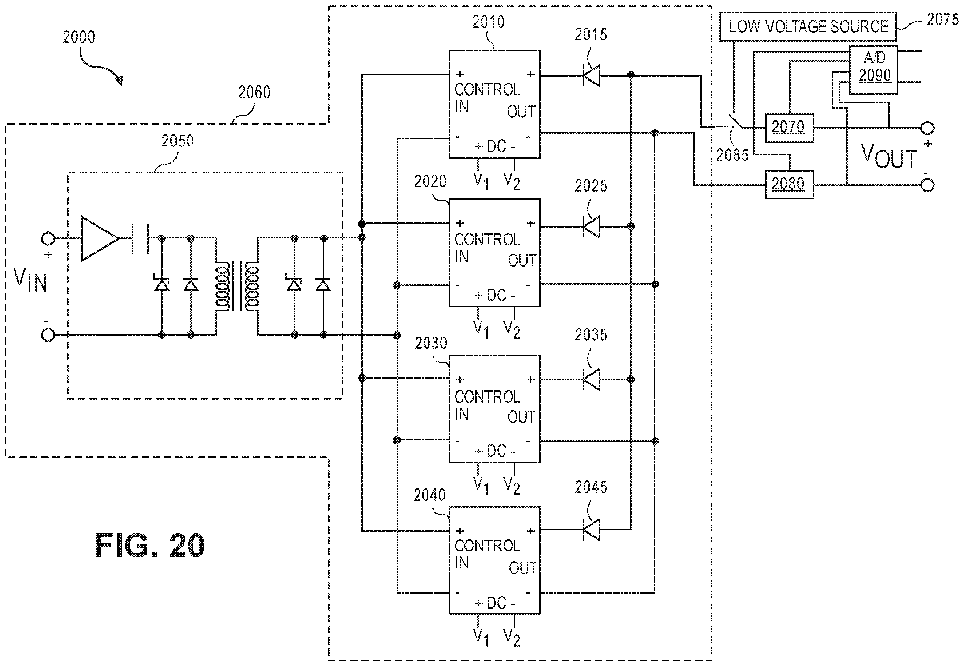

FIG. 20 illustrates an alternative pulse generator circuit 2000 which may be used inside nsPEF system 100 of FIG. 1. This pulse generator is similar to pulse generator circuit 700, described above with reference to FIG. 7. Pulse generator circuit 2000 generates output signals across output port Vout.

Pulse generator circuit 2000 includes multiple panels or pulse generator circuits 2010, 2020, 2030, and 2040, which may respectively be similar or identical to pulse generator circuits 710, 720, 730, and 740, described above with reference to FIG. 7. In this embodiment, four pulse generator circuits are used. In alternative embodiments, fewer or more pulse generator circuits are used. For example, in some embodiments, 2, 3, 5, 6, 7, 8, 9, 10 or another number of pulse generator circuits are used.

Pulse generator circuit 2000 also includes diodes 2015, 2025, 2035, and 2045, which are similar or identical to diodes 715, 725, 735, and 745, described above with reference to FIG. 7. Pulse generator circuit 2000 also includes driver 2050, which may be similar or identical to driver 750, described above with reference to FIG. 7.

Pulse generator circuits 2010, 2020, 2030, and 2040, diodes 2015, 2025, 2035, and 2045, and driver 2050 collectively form pulse generator circuit 2060 and collectively operate similarly or identically to pulse generator circuits 710, 720, 730, and 740, diodes 715, 725, 735, and 745, and driver 750, described above with reference to FIG. 7. In some embodiments, pulse generator circuit 2060 includes circuitry similar or identical to that described elsewhere herein, such that pulse generator circuit 2060 may be discharged with a discharge circuit.

Pulse generator 2000 also includes, or in some embodiments is connected to, analog-to-digital converter 2090. Furthermore, pulse generator 2000 additionally or alternatively includes, or in some embodiments is connected to, current monitors 2070 and 2080. The other pulse generator circuits discussed herein may similarly include or be connected to an analog to digital converter such as analog to digital converter 2090. Likewise, the other pulse generator circuits discussed herein may similarly include or be connected to current monitors such as current monitors 2070 and 2080.

In this embodiment, analog-to-digital (A/D) converter 2090 includes a first channel having inputs which are respectively connected to the positive (+) and negative (-) voltage output terminals of pulse generator 2000. In some embodiments, a first low input impedance differential buffer (not shown) is connected to the positive (+) and negative (-) voltage output terminals of pulse generator 2000, and drives the inputs of analog-to-digital converter 2090. In some embodiments, a probe, such as a Tektronix P6015A Passive High Voltage Probe (not shown) is connected to the positive (+) and negative (-) voltage output terminals of pulse generator 2000, and drives the inputs of analog-to-digital converter 2090.

In some embodiments, only the positive (+) voltage output terminal is connected to analog-to-digital converter 2090. In some embodiments, the positive (+) voltage output terminal is connected to analog-to-digital converter 2090 through a voltage divider. In such embodiments, the voltage at the positive (+) voltage output terminal is ground referenced, and the ground is also connected to analog-to-digital converter 2090. For example, the positive (+) voltage output terminal is ground referenced if the negative (-) voltage output terminal of pulse generator 2000 is at the ground voltage.

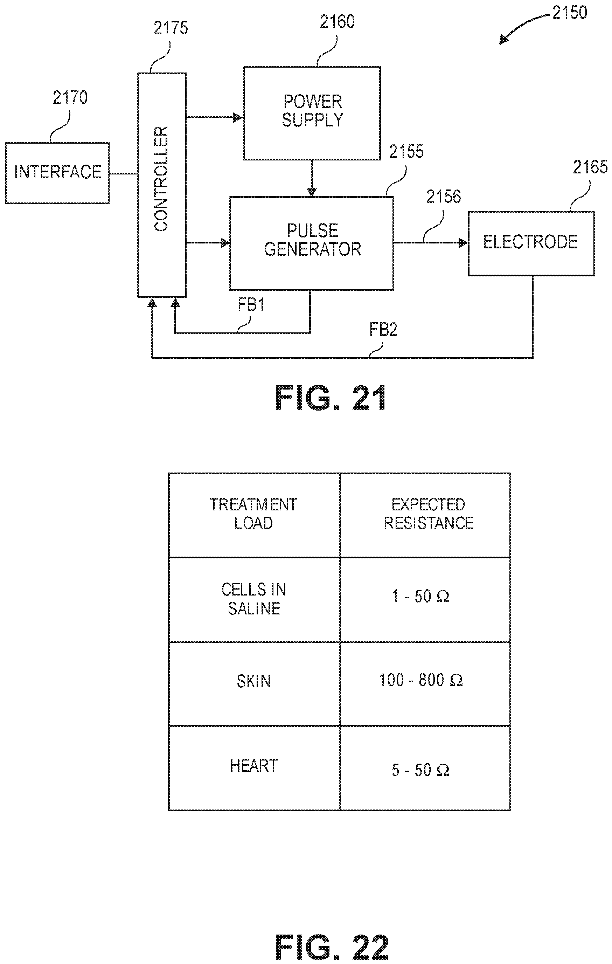

In addition, analog-to-digital converter 2090 is configured to generate a first digital output representing the voltage difference between the positive (+) and negative (-) voltage output terminals of pulse generator 2000. When used in the nsPEF treatment system 2150 of FIG. 21, the first digital output may be used as a feedback signal for controller 2175 of the nsPEF treatment system 2150 of FIG. 21, discussed below. In some embodiments, analog-to-digital converter 2090 generates the first digital output based on either, but not both, of the voltages at the positive (+) and negative (-) voltage output terminals.

In this embodiment, analog-to-digital converter 2090 also includes a second channel having inputs which are respectively connected to the current monitors 2070 and 2080, and the current monitors 2070 and 2080 are respectively connected to the positive (+) and negative (-) voltage output terminals of pulse generator 2000. In some embodiments, a second low input impedance differential buffer (not shown) is connected to the current monitors 2070 and 2080, and drives the inputs of analog-to-digital converter 2090.

In addition, analog-to-digital converter 2090 is configured to generate a second digital output representing the current difference between the currents flowing through positive (+) and negative (-) voltage output terminals of pulse generator 2000. When used in the nsPEF treatment system 2150 of FIG. 21, discussed below, the second digital output may be used as a feedback signal for controller 2175. In some embodiments, analog-to-digital converter 2090 generates the second digital output based on either, but not both, of inputs from the current monitors 2070 and 2080.

In some embodiments, current monitors 2070 and 2080 each include a sense resistor and an amplifier. The sense resistor is configured to generate a voltage response of the current flowing therethrough, and the amplifier generates an input for the analog-to-digital converter based on the voltage across the sense resistor.

In some embodiments, current monitors 2070 and 2080 include a current monitor, such as a Pearson Current Monitor 2878, which generates a voltage in response to a sensed current.

In some embodiments, pulse generator 2000 generates either, but not both, of the first and second digital outputs. In some embodiments, one or more single channel analog-to-digital converters are used instead of or in addition to analog-to-digital converter 2090.

In some embodiments, only single current monitor is used. The single current monitor may monitor the current of either of the positive (+) and negative (-) voltage output terminals of pulse generator 2000.

Pulse generator circuit 2000 also includes low voltage source 2075 and switch 2085.

Low voltage source 2075 may be a voltage source which is separate and independent from pulse generator circuit 2060. Low voltage source 2075 may be configured to generate a voltage which may be selectively output across output port Vout according to the state of switch 2085. The voltage level of the voltage generated by low voltage source 2075 may be less than the voltage level of the voltage generated by pulse generator circuit 2060.

In some embodiments, low voltage source 2075 is similar or identical to pulse generator circuit 2060, and is configured to deliver an nsPEF pulse having a voltage which is less than the voltage of the nsPEF pulse delivered by pulse generator circuit 2060. In alternative embodiments, low voltage source 2075 is similar or identical to any of the other pulse generator circuits described herein or may be another voltage source circuit, such as a different pulse generator circuit. In some embodiments, low voltage source 2075 is not used, and pulse generator circuit 2060 is selectively charged to different voltages to achieve both the high and low voltages discussed herein.

In alternative embodiments, low voltage source 2075 is another type of voltage source. For example, low voltage source 2075 may be a constant DC power supply. In such embodiments, the duration of a voltage delivered to output port Vout from the low voltage source 2075 is determined by the duration of the state of switch 2085 causing low voltage source 2075 to be electrically connected with the positive terminal of output port Vout.

FIG. 21 is a block diagram of a nsPEF treatment system 2150, which has characteristics similar to or identical to those of nsPEF system 100 illustrated in FIG. 1. NsPEF treatment system 2150 includes pulse generator 2155, power supply 2160, electrode 2165, interface 2170, and controller 2175.

Pulse generator 2155 may be similar or identical to any of the pulse generator circuits discussed herein. For example, pulse generator 2155 may be configured to generate pulses having a voltage level corresponding with power voltages received from power supply 2160 and having pulse widths and other characteristics corresponding with control signals received from controller 2175. In alternative embodiments, other pulse generator circuits may be used.

Electrode 2165 may be similar or identical to any of the electrodes discussed herein. For example, electrode 2165 may be similar or identical to electrodes 300 and 400 discussed above with reference to FIGs. 3 and 4. Electrode 2165 is configured to receive nsPEF pulses generated by pulse generator 2155 from conductor 2156 and is configured to deliver nsPEF pulses to a patient undergoing therapeutic nsPEF treatment. In alternative embodiments, other therapeutic electrodes may be used, for example, some embodiment use one or more of the electrodes discussed in U.S. Application No. 15/269,273, filed September 19, 2016, titled "HIGH VOLTAGE CONNECTORS FOR PUSLE GENERATORS," and/or in U.S. Application No. 62/33,270, filed May 16, 2016, titled "PULSE APPLICATOR".

Power supply 2160 is configured to provide power voltages to pulse generator 2155. For example, in embodiments where pulse generator 2155 is similar to pulse generator circuit 2000 illustrated in FIG. 20, power supply 2160 may be configured to provide power voltages corresponding with power voltages V1 and V2 of pulse generator circuit 700. In some embodiments, power supply 2160 generates and provides power voltages which have a voltage level corresponding with a control signal from controller 2175.

Interface 2170 is configured to receive input from a user identifying various parameters and characteristics of the nsPEF pulses to be applied to the patient. For example, interface 2170 may be configured to receive input identifying or specifying values for one or more characteristics of one or more nsPEF pulses to be applied to the patient. For example, the characteristics may include one or more of an amplitude, a polarity, a width, a rise time, and a fall time of one or more nsPEF pulses to be applied to the patient. Additionally or alternatively, the characteristics may include one or more of a frequency and a pulse quantity of a sequence of nsPEF pulses to be applied to the patient. Furthermore, the characteristics may additionally or alternatively include a result of the nsPEF pulses to be applied to the patient, such as a maximum temperature for the treated tissue of the patient. Other characteristics may additionally or alternatively be identified or specified by the received input.

In addition, interface 2170 is configured to communicate the characteristics identified or specified by the received input to controller 2175.

Controller 2175 is configured to generate and provide one or more control signals to pulse generator 2155 and to power supply 2160 based at least partly on the communicated characteristics received from interface 2170. Additionally, pulse generator 2155, power supply 2160, and electrode 2165 are collectively configured to, in response to the control signals from controller 2175, generate nsPEF pulses having characteristics corresponding with the control signals.

In this embodiment, one or both of pulse generator 2155 and electrode 2165 are configured to generate feedback signals FB 1 and FB2 corresponding with or representing measured parametric characteristics of the nsPEF pulses applied to the patient. In some embodiments, the parametric characteristics of the nsPEF pulses represented by the feedback signals FB 1 and FB2 include one or more of an amplitude, a polarity, a width, a rise time, and a fall time of the nsPEF pulses. Additionally or alternatively, the parametric characteristics may include a frequency of a sequence of nsPEF pulses. Furthermore, the parametric characteristics may additionally or alternatively include a temperature of the treated tissue of the patient, or an impedance of the load. The feedback signals FB 1 and FB2 may correspond or represent other measured parametric characteristics of one or more of the nsPEF pulses applied to the patient, the patient, the environment, and the nsPEF treatment system 2150. In alternative embodiments, only one of feedback signals FB 1 and FB2 are generated. In some embodiments, neither of feedback signals FB 1 and FB2 are generated.

In some embodiments, controller 2175, power supply 2160, pulse generator 2155, and electrode 2165 collectively form a feedback loop which causes one or more parametric characteristics of the nsPEF pulses applied to the patient to have measured values substantially equal (e.g. within 10%, 5%, 3%, 2%, or 1%) to the values of corresponding characteristics identified in the input received by interface 2170.

For example, interface 2170 may receive input specifying a value of 15kV for an amplitude of the nsPEF pulses applied to the patient. In addition, the controller 2175 may be configured to, in response to a feedback signal FB2 from electrode 2165 or a feedback signal FB1 from pulse generator 2155 indicating that the measured amplitude of the nsPEF pulses applied to the patient is less than (or greater than) 15kV, change a control signal provided to power supply 2160. In response to the changed control signal, power supply 2160 may be configured to increase (or decrease) the voltage of power signals provided to pulse generator 2155 such that the amplitude of the nsPEF pulses generated and applied to the patient increases (or decreases) to or toward 15kV.

Similarly, interface 2170 may receive input specifying a value of 150ns for a pulse width of the nsPEF pulses applied to the patient. The controller 2175 may be configured to, in response to a feedback signal FB2 from electrode 2165 or a feedback signal FB1 from pulse generator 2155 indicating that the measured pulse width of the nsPEF pulses applied to the patient is greater than (or less than) 150ns, change a control signal provided to pulse generator 2155. In response to the changed control signal, pulse generator 2155 may be configured to generate and apply to the patient nsPEF pulses having decreased (or increased) pulse width. As a result, the feedback signal FB 1 or FB2 causes the controller 2175 to generate control signals which cause the pulse generator 2155 to generate and apply nsPEF pulses having pulse widths decreased (or increased) to or toward 150ns.

In some embodiments, the feedback loop is controlled using a Proportional-Integral-Derivative (PID) method. For example, controller 2175 may be configured to continuously or substantially continuously calculate an error value as the difference between a desired value perceived at interface 2170 and a corresponding measured parameter. In addition, controller 2175 may be configured to continuously or substantially continuously calculate the control signals as a sum of one or more of: a first constant times the error signal, a second constant times an integral of the error signal, and a third constant times a derivative of the error signal.

In some embodiments, the feedback loop is controlled using a lookup table to determine a next value based on a measured value. In some embodiments, the feedback loop is controlled by reducing or increasing a value by a fixed amount or step size based on a determination of whether a measured value is greater than or less than a threshold.

Various pulse generator circuits have been discussed herein and are capable of delivering voltage pulses of differing durations and differing voltage levels. For example, pulse generator circuit 2000 of FIG. 20 is configured to deliver pulses of differing voltage levels by multiplexing between two separate sources. Additionally, pulse generator circuit 1800 of FIG. 18 is configured to deliver pulses of differing voltage levels by charging and/or discharging storage capacitors to different voltage levels.

A pulse generator circuit having the ability to deliver voltages of different voltage levels is particularly advantageous. For example, such a pulse generator circuit may be used to deliver pulses of a first voltage level for therapeutic or experimental use and to deliver pulses of a second voltage level for other uses. For example, such a pulse generator circuit may be used to deliver therapeutic or experimental pulses having a voltage level of 5 kV, and may also be used to deliver pulses of a significantly lower voltage level for system diagnostic or system test purposes. For example, diagnostic or test pulses may be delivered with a voltage level of 1 kV, 0.5 kV, 250 V, 100V, 50V, 25V, 10V, 5V, 3V, 1V, or another voltage level.

The diagnostic or test pulses may be used to verify that the pulse generator system is working properly, and/or that electrodes are properly placed. For example, after electrodes are placed on opposite sides of a tissue to be treated, the tissue to be treated presents an electrical load to the pulse generator. Because the electrical properties of the tissue are known, the expected resistance of the load is known. As part of a system diagnostic or test routine, for example, to increase system safety, one or more low voltage pulses may be delivered to the load while measuring the current delivered to the load during each pulse. Based on the voltage level of the pulse delivered to the load and the measured current, an electrical resistance of the load can be calculated. The calculated electrical resistance of the load may be compared with an expected value or range of values determined based on the type of tissue constituting the load. The pulse generator system is configured to determine whether the pulse generator system passed or failed the diagnostic or test routine based on whether the calculated electrical resistance falls within the expected range of values.

In some embodiments, to pass, the pulse generator system is required to generate multiple consecutive impedance measurements falling within the expected range of values. For example, to pass, the pulse generator system is required to generate 2, 3, 4, 5, or more consecutive impedance measurements falling within the expected range of values.

In some embodiments, a result of the system diagnostic or test is indicated on an electronic display interface. For example, passing and failing results may be indicated by distinct indicators. In some embodiments, an indication of measured impedance is displayed. In some embodiments, an indication is displayed showing whether the system failed as a result of the measured impedance being greater than the expected range or as a result of the measured impedance being less than the expected range.

In some embodiments, the system automatically responds to the result of the system diagnostic. For example, a failing result may cause the system to stop delivering treatment pulses and/or to generate a report. Similarly, a passing result may cause the system to start or continue delivering treatment pulses.

The duration of the low voltage pulse is not limited, and may, for example, be 20 ns, 50 ns, 100 ns, 200 ns, 500 ns, 1000 ns, 2000 ns, 5000 ns, or another duration. Multiple pulses may be delivered, for example, at a substantially constant frequency. The frequency is not limited, and may, for example, be 0.1 Hz, 0.2 Hz, 0.5 Hz, 1 Hz, 2 Hz, 5 Hz, 10 Hz, 20 Hz, 50 Hz, or another frequency.

In some embodiments, the duration of the low voltage pulse is dependent on the time between an adjacent pair of treatment pulses in a series of treatment pulses. For example, the duration of the low voltage pulse between adjacent treatment pulses may be a predetermined percentage of the time between the adjacent treatment pulses. In some embodiments, the duration of the low voltage pulse between adjacent treatment pulses may be equal to the time between the adjacent treatment pulses minus a fixed time margin.

A system diagnostic or test routine may be performed, for example, in response to an indication from an operator of the pulse generator. For example, a doctor may press a button and, in response to the pressed button, the pulse generator may automatically perform a diagnostic or test routine. In some embodiments, for example, a system diagnostic or test routine may be performed in response to an indication from a user to begin a treatment. In response to the indication, prior to delivering treatment pulses, and in some embodiments, prior to charging the pulse generator, the pulse generator system may perform a system diagnostic or test routine. In response to the system passing the diagnostic or test routine, the pulse generator may charge the pulse generator, if necessary, and begin treatment. In contrast, in response to the system not passing the diagnostic or test routine, the pulse generator may not begin treatment or may not even charge the pulse generator, despite the indication from the user. In some embodiments, the indications may be generated in response to a user interacting with a graphical user interface on a display.

In some embodiments, a system diagnostic test or routine may be performed as part of an initialization routine performed by the pulse generator system.

In some embodiments, a system diagnostic test or routine may be interrupted in response to an indication from a user.

In some embodiments, multiple impedance measurements (e.g., 2 or more) are taken, and a difference in impedance values may be calculated. The calculated impedance difference may be compared with a threshold, and the system diagnostic test or routine may be determined to fail if the difference is greater than the threshold.

In some embodiments, multiple impedance measurements are taken during a treatment session, and a difference in impedance values may be calculated and compared with a threshold. The session may be interrupted as a result of the difference being either greater than the threshold or less than the threshold. Alternatively, an electrical parameter of the applied pulses of the treatment session may be changed. For example, in response to the difference between the impedance values being greater than the threshold or being less than the threshold, any of the voltage, the frequency, the duration, and another parameter of the treatment pulses may be increased or decreased. Of course, if the impedance difference (for those embodiments where a plurality of impedance measurements are taken) is within the threshold or permitted range, then the treatment session may continue without a need to change parameters. The same applies for those embodiments where a single impedance measurement is taken.

FIG. 22 is a table of expected impedance ranges for certain treatment loads. To determine whether the pulse generator system passes or fails a diagnostic or test routine, one or more calculated impedance values may be compared with expected values or ranges associated with load tissue type. The information of FIG. 22 may be uploaded, entered, or programmed into a pulse generator system so that the information is accessible in order for the system to compare calculated impedance values with the expected values or ranges of FIG. 22. The treatment loads and impedance ranges shown in FIG. 22 are exemplary only, and other treatment loads and impedance ranges may be used. In some embodiments, the expected impedance ranges are dependent on a type of electrode used. For example, the system may determine an expected impedance range based on an indication of the electrode or type of electrode being used.

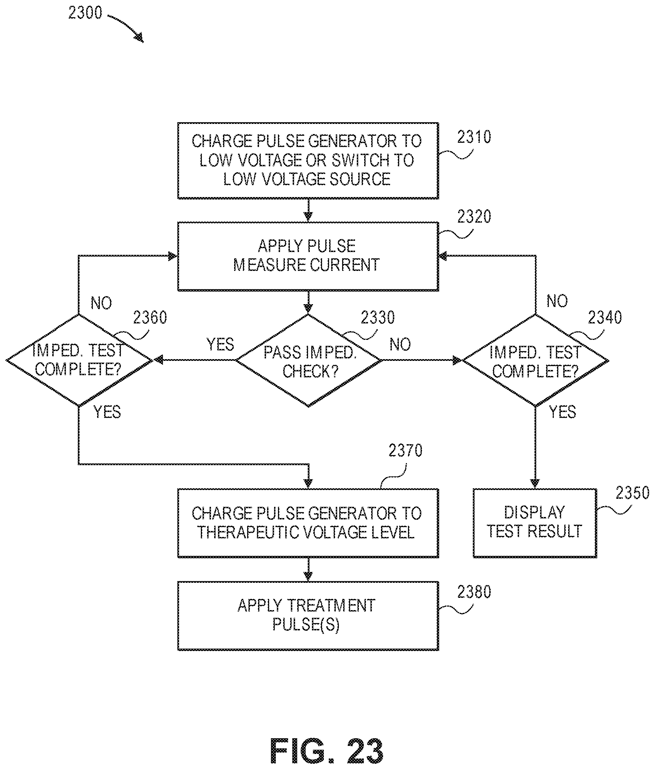

FIG. 23 is a flowchart diagram illustrating an example of a method 2300 of testing a pulse generator system setup status. The method 2300 may, for example, be performed by a treatment system, such as system 2150 of FIG. 21.

At 2310, the controller of the system configures the pulse generator of the system to deliver low voltage pulses, where the voltage level of the low voltage pulses is less than a voltage level of pulses used for treatment. For example, the controller may charge or discharge storage capacitors to a charge voltage corresponding with the voltage level of the low voltage pulses. Alternatively, the controller may selectively connect the output of the pulse generator to a low voltage source with a switch.

At 2320, the controller causes the pulse generator to apply a voltage pulse to an electrode connected to a load. The controller also receives a signal indicating the amount of current applied to the load while the voltage pulse is applied to the load.

At 2330, the controller calculates a load impedance, for example by mathematically dividing the voltage level of the voltage pulse by the measured current. The controller also compares the calculated load impedance with an expected impedance or expected impedance range determined based on a treatment load type, for example, previously programmed into the controller. Based on the result of the comparison, the controller determines whether the calculated load impedance falls within the expected impedance range.

If the calculated load impedance does not fall within the expected impedance range or is within a threshold of the expected impedance, at 2340, the controller determines whether to continue the test, based, for example, on a time limit condition or on whether a quantity of impedance measurements has been taken. In some embodiments, a single calculated load impedance falling outside the expected impedance range or is not within the threshold of the expected impedance, as determined at 2330, causes the method to proceed to 2350.

If the controller determines that the test is finished, at 2350, an indication of the failing result of the system test is displayed on an interface. If the controller determines, at 2340, to continue the test, at 2320, another pulse is delivered, as described above.

If the calculated load impedance is determined, at 2330, to fall within the expected impedance range or is within the threshold of the expected impedance, at 2360, the controller determines whether to continue the test, based, for example, on a time limit condition or on whether a quantity of impedance measurements has been taken. In some embodiments, a single calculated load impedance falling within the expected impedance range or is within the threshold of the expected impedance, as determined at 2330, causes the method to proceed to 2370.

At 2370, the controller may configure the pulse generator of the system to deliver treatment or therapeutic pulses, where the voltage level of the treatment (or therapeutic) pulses is greater than a voltage level of diagnostic or test pulses used for determining the load impedance at 2320. For example, at 2370, the controller may charge or discharge storage capacitors to a therapeutic charge voltage corresponding with the voltage level of the treatment pulses.

At 2380, the controller causes the pulse generator to deliver treatment pulses to the load through the electrodes.

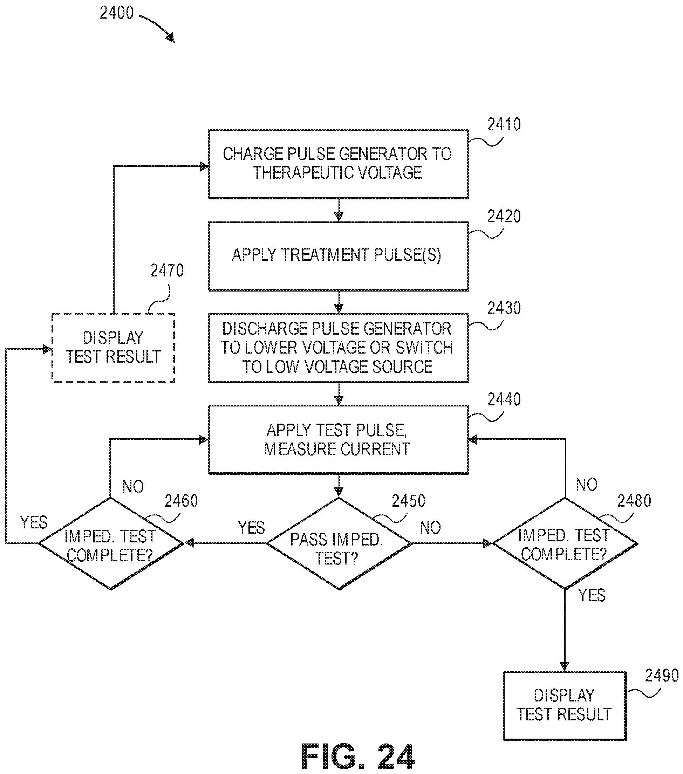

FIG. 24 is a flowchart diagram illustrating an example of another method 2400 of testing a pulse generator system setup status. The method 2400 may, for example, be performed by a treatment system, such as system 2150 of FIG. 21.

At 2410, the controller of the system configures the pulse generator of the system to deliver treatment (or therapeutic) pulses, where the voltage level of the treatment pulses is greater than a voltage level of pulses used for determining the load impedance at 2430, discussed below. For example, the controller may charge or discharge storage capacitors to a voltage corresponding with the voltage level of the treatment pulses.

At 2420, the controller causes the pulse generator to deliver treatment pulses to a load through electrodes of the system.

At 2430, the controller of the system configures the pulse generator of the system to deliver low voltage pulses, where the voltage level of the low voltage pulses is less than the voltage level of pulses used for treatment. For example, the controller may charge or discharge storage capacitors to a voltage corresponding with the voltage level of the low voltage pulses. Alternatively, the controller may selectively connect the output of the pulse generator to a low voltage source with a switch.

At 2440, the controller causes the pulse generator to apply a voltage pulse, e.g., diagnostic or test pulse, to the electrode connected to the load. The controller also receives a signal indicating the amount of current applied to the load while the voltage pulse is applied to the load.

At 2450, the controller calculates a load impedance, for example by mathematically dividing the voltage level of the voltage pulse by the measured current. The controller also compares the calculated load impedance with an expected impedance or impedance range determined, for example, based on a treatment load type previously programmed into the controller. Based on the result of the comparison, the controller determines whether the calculated load impedance falls within the expected impedance range or is within the threshold of the expected impedance.

If the calculated load impedance does not fall within the expected impedance range or is not within the threshold of the expected impedance, at 2480, the controller determines whether to continue the test, based, for example, on a time limit condition or on whether a quantity of impedance measurements has been taken. In some embodiments, a single calculated load impedance falling outside the expected impedance range or not within the threshold of the expected impedance, as determined at 2450, causes the method to proceed to 2490.

If the controller determines, at 2480, that the test is finished, at 2490, an indication of the failing result of the system test may be displayed on an interface and treatment is stopped, or at least interrupted to address any issues. If the controller determines to continue the test, at 2440, another pulse is delivered, as described above.

If the calculated load impedance is determined, at 2450, to fall within the expected impedance range or to be within the threshold of the expected impedance, at 2460, the controller determines whether to continue the test, based, for example, on a time limit condition or on whether a quantity of impedance measurements has been taken. In some embodiments, a single calculated load impedance falling within the expected impedance range or within the threshold of the expected impedance, as determined at 2450, causes the method to proceed to optional 2470, where an indication of the passing result of the system test may be displayed on an interface. If optional 2470 is not used, at 2410, the controller of the system configures the pulse generator of the system to deliver treatment pulses, as discussed above.

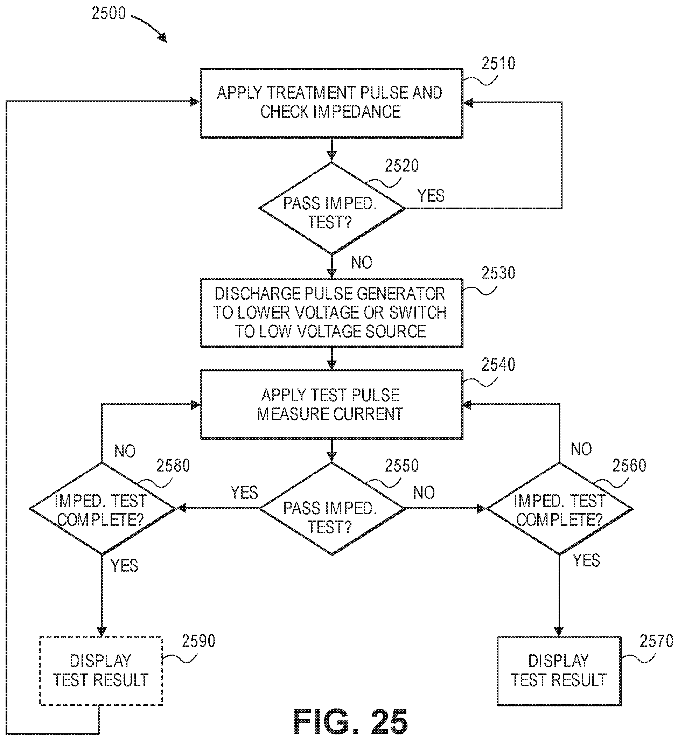

FIG. 25 is a flowchart diagram illustrating an example of a method 2500 of testing a pulse generator system setup status. The method 2500 may, for example, be performed by a treatment system, such as system 2150 of FIG. 21.

At 2510, the controller causes the pulse generator to apply a treatment voltage pulse to an electrode connected to a load. The controller also receives a signal indicating the amount of current applied to the load while the treatment voltage pulse is applied to the load. In addition, the controller calculates a load impedance, for example by mathematically dividing the voltage level of the treatment voltage pulse by the measured current.

At 2520, the controller also compares the calculated load impedance with an expected impedance or impedance range determined based on a treatment load type previously programmed into the controller. Based on the result of the comparison, the controller determines whether the calculated load impedance falls within the expected impedance range or is within the threshold of the expected impedance.

If the calculated load impedance is within the expected impedance range or is within the threshold of the expected impedance, at 2510, the controller causes the pulse generator to apply a treatment voltage pulse to the electrode. If the calculated load impedance does not fall within the expected impedance range or is not within the threshold of the expected impedance, at 2530, in some embodiments the controller configures the pulse generator of the system to deliver low voltage pulses (e.g., test pulses), where the voltage level of the low voltage pulses is less than a voltage level of pulses used for treatment. For example, the controller may charge or discharge storage capacitors to a voltage corresponding with the voltage level of the low voltage pulses. Alternatively, the controller may selectively connect the output of the pulse generator to a low voltage source with a switch.

At 2540, the controller causes the pulse generator to apply a voltage pulse to the electrode, where the voltage level of the applied voltage pulse is less than the voltage level of the treatment pulse. The controller also receives a signal indicating the amount of current applied to the load while the low voltage pulse is applied to the load.

At 2550, the controller calculates a load impedance, for example by mathematically dividing the voltage level of the low voltage pulse by the measured current. The controller also compares the calculated load impedance with an expected impedance or impedance range determined based on a treatment load type previously programmed into the controller. Based on the result of the comparison, the controller determines whether the calculated load impedance falls within the expected impedance range or is within the threshold of the expected impedance.

If the calculated load impedance does not fall within the expected impedance range or is not within the threshold of the expected impedance, at 2560, the controller determines whether to continue the test, based, for example, on a time limit condition or on whether a quantity of impedance measurements has been taken. In some embodiments, a single calculated load impedance falling outside the expected impedance range or not within the threshold of the expected impedance, as determined at 2550, causes the method to proceed to 2570.

If the controller determines that the test is finished, at 2570, an indication of the failing result of the system test may be displayed on an interface. If the controller determines to continue the test, at 2540, another pulse is delivered, as described above.

If the calculated load impedance is determined, at 2550, to be within the expected impedance range or to be within the threshold of the expected impedance, at 2580, the controller determines whether to continue the test, based, for example, on whether a time or on whether a quantity of impedance measurements has been taken. In some embodiments, a single calculated load impedance falling within the expected impedance range or within the threshold of the expected impedance, as determined at 2550, causes the method to proceed to 2590.

If the controller determines that the test is finished, at 2590, an indication of the passing result of the system test may be optionally displayed on an interface. If the controller determines to continue the treatment, the pulse generator is charged to a treatment voltage and the process starts again by applying a treatment pulse at 2510.

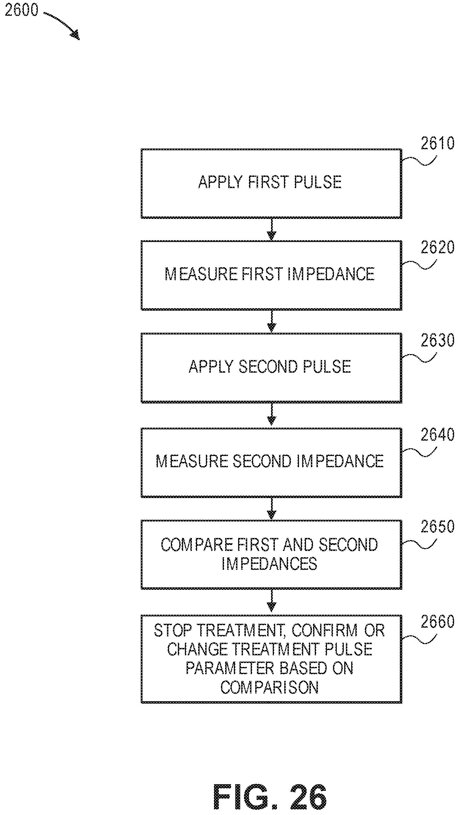

FIG. 26 is a flowchart diagram illustrating an example of a method 2600, not part of the claimed invention. The method 2600 may, for example, be performed by a treatment system, such as system 2150 of FIG. 21.

At 2610, a first pulse is applied to a patient. The first pulse may be any type of pulse. For example, the first pulse may be high voltage or low voltage. It may be a therapeutic pulse or it may be a test pulse. The voltage, duration, frequency, voltage shape, and any other electrical parameter of the pulse are not limited. The first pulse may be similar or identical to any other pulse described herein.

At 2620, a first impedance is measured based on the voltage and current delivered to the patient. The method of measurement is not limited, and may, for example, be similar or identical to any of the impedance measurement methods discussed herein. The first impedance may be stored in a memory.

At 2630, a second pulse is applied to a patient. The second pulse may be any type of pulse. For example, the second pulse may be high voltage or low voltage. It may be a therapeutic pulse or it may be a test pulse. The voltage, duration, frequency, voltage shape, and any other parameter of the second pulse are not limited. The second pulse may be similar or identical to any other pulse described herein.

At 2640, a second impedance is measured based on the voltage and current delivered to the patient. The method of measurement is not limited, and may, for example, be similar or identical to any of the impedance measurement methods discussed herein. The second impedance may be stored in a memory.

In some embodiments, one of the first and second pulses is a therapeutic pulse, for example having a relatively higher voltage compared to the other of the first and second pulses, which is a test pulse.

The temporal and sequential relationship between the first and second pulses is not limited. In some embodiments, the first and second pulses are adjacent to one another in a series of applied pulses. In some embodiments, the first pulse is an initial pulse in a series of applied pulses, and the second pulse is a subsequent adjacent or non-adjacent applied pulse in the series of applied pulses.

In some embodiments, the first impedance represents an initial or benchmark impedance of the load, and the second impedance represents an impedance of the load after one or more treatment pulses.

At 2650, a difference between the first and second impedances is determined.

At 2660, a determination is made regarding further treatment based on the difference between the first and second impedances. For example, in response to a difference between the first and second impedances being greater than a threshold or outside of a threshold range, treatment may be stopped. In some embodiments, in response to a difference between the first and second impedances being less than a threshold or within a threshold range, treatment may be stopped. In some embodiments, the difference being greater than a threshold, or outside of a threshold range, or less than a threshold, or within a threshold range may indicate a problem with the treatment. In some embodiments, the difference being greater than a threshold, or outside of a threshold range may indicate that the treatment is completed, and can or should be stopped.

In some embodiments, the impedance of the load is expected to change over the course of a treatment session. For example, the impedance of the load may be expected to track a known impedance profile. In response to a deviation from the profile being greater than a threshold, treatment may be stopped. In some embodiments, in response to a deviation from the profile being greater than a threshold, an electrical parameter of the treatment pulses may be modified. For example, in response to the deviation from the profile being greater than the threshold or being less than the threshold, any of the voltage, the frequency, the duration, the voltage shape, and another parameter of the treatment pulses may be increased, decreased, or otherwise changed. Similarly, in response to the impedance profile being within a threshold or acceptable range or deviation from the profile being within a threshold or acceptable range, one or more parameters of the treatment pulses may be confirmed without modification.

Applying nsPEF to a tumor sufficient to stimulate apoptosis includes at least the electrical characteristics found experimentally. For example, a 100 ns long pulse with a 20 ns rise time to 30 kV/cm (kilovolts per centimeter) at 1 to 7 pulses per second (pps) for 500 to 2000 pulses has been found to be sufficient to stimulate apoptosis, depending on the tumor type. Pulsed electric fields of at least 20 kV/cm have been shown to be effective. A number of pulses greater than 50 pulses has also been shown to be effective. Current values between 12 A and 60 A resulted, depending on the electrode type and skin resistance.

The embodiments of pulse generators described herein have many uses. In one nonlimiting example, cancer that has metastasized through a subject's bloodstream may be treated using nsPEF's immune stimulation properties. For treatment, circulating tumor cells (CTCs) are isolated from the bloodstream and amassed in vial, test tube, or other suitable in vitro environment. In some cases, there may only be a few (e.g., 5, 10), tumor cells that are collected and amassed. Through this mass, an nsPEF electric field is applied in order to treat the cells. This may cause calreticulin or one or more other damage-associated molecular patterns (DAMPs) to be expressed on the surface membranes of the tumor cells. The tumor cells may then be introduced back into the subject's bloodstream by injection, infusion, or otherwise.

In an alternative embodiment, single CTCs may also be isolated from the bloodstream, and each tumor cell treated individually. An automated system that captures CTCs in whole blood using iron nanoparticles coated with a polymer layer carrying biotin analogues and conjugated with antibodies for capturing CTCs can automatically capture the tumor cells, and a magnet and or centrifuge can separate them. After separation from the antibodies, the CTCs may be treated with nsPEF through a small capillary and then reintroduced to the patient's bloodstream.

While examples in the application discuss human and murine subjects, the treatment of other animals is contemplated. Agricultural animals, such as horses and cows, or racing animals, such as horses, may be treated. Companion animals, such as cats and dogs, may find special use with the treatments described herein. It may be difficult for a veterinarian to remove many tumors from a small animal, and cancers may be caught relatively late because the animals cannot communicate their advancing pain. Further, the risk inherent in reinjecting tumor cells-albeit treated tumor cells-may be worth the potential benefits of potentially halting a metastasized cancer in a loved pet.

The methods of the impedance check of the present disclosure can be used with pulse generators, for example, pulse generators for the treatment of any type of tumor, whether characterized as malignant, benign, soft tissue, or solid, and cancers of all stages and grades including pre- and post-metastatic cancers. Examples of different types of cancer include, but are not limited to, digestive and gastrointestinal cancers such as gastric cancer (e.g., stomach cancer), colorectal cancer, gastrointestinal stromal tumors, gastrointestinal carcinoid tumors, colon cancer, rectal cancer, anal cancer, bile duct cancer, small intestine cancer, and esophageal cancer; breast cancer; lung cancer; gallbladder cancer; liver cancer; pancreatic cancer; appendix cancer; prostate cancer, ovarian cancer; renal cancer (e.g., renal cell carcinoma); cancer of the central nervous system; skin cancer (e.g., melanoma); lymphomas; gliomas; choriocarcinomas; head and neck cancers; osteogenic sarcomas; and blood cancers.

Electrical characteristics of nsPEF treatments can be adjusted based on a size and/or a type of a tumor. Types of tumors may include tumors of different regions of the body, such as the cancerous tumors described above.

It is understood that the various embodiments described herein are by way of example only, and are not intended to limit the scope of the invention. For example, many of the materials and structures described herein may be substituted with other materials and structures without deviating from the spirit of the invention. The present invention as claimed may therefore include variations from the particular examples and preferred embodiments described herein, as will be apparent to one of skill in the art. It is understood that various theories as to why the invention works are not intended to be limiting.

The above description is illustrative and is not restrictive. Many variations of the invention will become apparent to those skilled in the art upon review of the disclosure. The scope of the invention should, therefore, be determined not with reference to the above description, but instead should be determined with reference to the pending claims.

As noted previously, all measurements, dimensions, and materials provided herein within the specification or within the figures are by way of example only.

A recitation of "a," "an," or "the" is intended to mean "one or more" unless specifically indicated to the contrary. Reference to a "first" component does not necessarily require that a second component be provided. Moreover reference to a "first" or a "second" component does not limit the referenced component to a particular location unless expressly stated.

All publications mentioned herein disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates, which may need to be independently confirmed.

## Claims

1. A therapeutic nsPEF pulse generator system (100, 2150), comprising:
an electrode (2165);
a pulse generator circuit (2155) electrically connected to the electrode and configured to deliver pulses to the electrode; and
a controller (2175) connected to the pulse generator circuit (2155) and configured to determine a voltage level of the pulses delivered to the electrode, wherein the controller is further configured to:
cause the pulse generator circuit (2155) to be charged to a first charge voltage,
cause the pulse generator circuit (2155) to deliver a first voltage pulse to a load at the first charge voltage,
cause the pulse generator (2155) to be discharged to a second charge voltage lower than the first charge voltage using a discharge circuit,
cause the pulse generator (2155) to deliver a second voltage pulse to the load through the electrode (2165) at the second charge voltage,
receive a signal indicating an impedance of the load,
compare the impedance with an expected impedance, and
at least partly in response to the comparison, determine
1) to deliver a third voltage pulse to the load, based at least in part on a determination that the pulse generator circuit (2155) passed a test based at least in part on the impedance being within a threshold range of the expected impedance; and
2) to not deliver a third voltage pulse to the load based at least in part on a determination that the impedance being outside the threshold range of the expected impedance,
wherein at least one of the first and the second voltage pulses is therapeutic to the load.

2. The system (100, 2150) of claim 1, wherein the expected impedance of the load is determined by the system (100, 2150) based on a type of electrode being used, or the expected impedance is uploaded, entered or programmed into the system.

3. The system (100, 2150) of claim 1, wherein the controller (2175) is further configured to determine the expected impedance based on an identification of the load, optionally wherein the expected impedance comprises an impedance range.

4. The system (100, 2150) of any one of claims 1-3, wherein the controller (2175) is configured to perform the test multiple times, and wherein determining to deliver the third voltage pulse is based at least in part on passing the test at least a number of consecutive times.

5. The system (100, 2150) of claim 1, wherein the controller (2175) is configured to perform the test multiple times and determine the test is complete based at least in part on a quantity of impedance measurements taken.

6. The system (100, 2150) of any one of claims 1-3, wherein the controller (2175) is configured to perform the test multiple times and determine the test is complete based at least in part on a time limit condition.

7. The system (100, 2150) of any one of claims 1-6, wherein the system (100, 2150) is configured to charge or discharge the pulse generator circuit (2155) to a third charge voltage.

8. The system (100, 2150) of any one of claims 1-7, wherein the system (100, 2150) is configured to display a graphical indication based on the comparison.

9. The system (100, 2150) of any one of claims 1-8, wherein the second voltage pulse is a low voltage test pulse and the first voltage pulse is a therapeutic treatment pulse.

10. The system (100, 2150) of any one of claims 1-8, further comprising a low voltage source.

11. The system (100, 2150) of any one of claims 1- 10, wherein the impedance of the load is determined by:
measuring a current, wherein the current is delivered to the load during the delivery of the second voltage pulse;
determining a voltage of the delivered second voltage pulse; and
calculating the impedance based on a voltage level of the second voltage pulse and on the measured current.

12. The system (100, 2150) of any one of claims 1-11, wherein the controller (2175) is further configured to cause the pulse generator circuit (2155) to deliver a third voltage pulse to the load and wherein delivering the third voltage pulse comprises changing a state of a switch so as to electrically disconnect the load from a first voltage pulse source and to connect the load to a second voltage pulse source.

13. The system (100, 2150) of any one of claims 1-12, wherein the controller is further configured to determine whether an impedance test is complete; and
based on the result of 1) a determination of whether the impedance test is complete and 2) the comparison of the impedance with the expected impedance, determine whether to apply a pulse of a test voltage level, or a pulse of a therapeutic voltage level, or to stop applying pulses.

14. The system (100, 2150) of any one of claims 1-13, wherein the controller (2175) is further configured to cause the pulse generator circuit (2155) to deliver a third voltage pulse to the load and wherein third voltage pulse is a treatment pulse.

15. A therapeutic nsPEF pulse generator system (100, 2150), comprising:
an electrode (2165);
a pulse generator circuit (2155) electrically connected to the electrode (2165) and configured to deliver voltage pulses to the electrode (2165); and
a controller (2175) connected to the pulse generator circuit (2155) and configured to determine a voltage level of the voltage pulses delivered to the electrode, wherein the controller (2175) is further configured to:
cause the pulse generator circuit (2155) to deliver a first voltage pulse to a load through the electrode;
determine a first impedance of the load at least based partly on the delivered first voltage pulse;
cause the pulse generator circuit (2155) to deliver a second voltage pulse to a load through the electrode;
determine a second impedance of the load based at least partly on the delivered second voltage pulse;
compare the first impedance with the second impedance; and
as a result of the comparison, discharging the pulse generator circuit (2155) to a second charge voltage lower than the first charge voltage using a discharge circuit to:
a) stop delivery of voltage pulses to the load, or
b) deliver a next voltage pulse to the load, wherein the next voltage pulse has an electrical parameter which is different from a corresponding electrical parameter of the first and second voltage pulses.

16. The system (100, 2150) of claim 15, wherein the controller (2175) is configured to stop delivery of voltage pulses to the load as a result of a difference between the first and second impedance being greater or less than a threshold or outside of a threshold range.

17. The system (100, 2150) of claims 15 or 16, wherein the first and the second voltage pulses are therapeutic pulses, or wherein the first and the second voltage pulses are test pulses.

18. The system (100, 2150) of any one of claims 15-17, wherein the voltage pulse having the different electrical parameter is delivered as a result of the comparison indicating a deviation from an impedance profile being greater than a threshold.

19. The system (100, 2150) of any one of claims 15-18, wherein the electrical parameter is one of a voltage, a frequency, a duration, and a voltage shape.

## Patentansprüche

1. Therapeutisches nsPEF-Impulsgeneratorsystem (100, 2150), umfassend:
eine Elektrode (2165); eine Impulsgeneratorschaltung (2155), die elektrisch mit der Elektrode verbunden und konfiguriert ist, um Impulse an die Elektrode zu liefern; und
eine Steuerung (2175), die mit der Impulsgeneratorschaltung (2155) verbunden und konfiguriert ist, um einen Spannungspegel der Impulse zu bestimmen, die an die Elektrode abgegeben werden, wobei die Steuerung ferner konfiguriert ist zum:
Bewirken, dass die Impulsgeneratorschaltung (2155) auf eine erste Ladespannung geladen wird,
Veranlassen der Impulsgeneratorschaltung (2155), einen ersten Spannungsimpuls an eine Last mit der ersten Ladespannung abzugeben,
Veranlassen, dass der Impulsgenerator (2155) auf eine zweite Ladespannung entladen wird, die niedriger als die erste Ladespannung ist, unter Verwendung einer Entladeschaltung,
Veranlassen, dass der Impulsgenerator (2155) einen zweiten Spannungsimpuls an die Last durch die Elektrode (2165) an der zweiten Ladespannung abgibt,
Empfangen eines Signals, das eine Impedanz der Last angibt,
Vergleichen der Impedanz mit einer erwarteten Impedanz und
mindestens teilweise als Reaktion auf den Vergleich, Bestimmen,
1) einen dritten Spannungsimpuls an die Last, mindestens teilweise basierend auf einer Bestimmung, dass die Impulsgeneratorschaltung (2155) einen Test besteht, der mindestens teilweise auf der Impedanz basiert, die innerhalb eines Schwellenbereichs der erwarteten Impedanz liegt, abzugeben; und
2) einen dritten Spannungsimpuls mindestens teilweise basierend auf einer Bestimmung, dass die Impedanz außerhalb des Schwellenbereichs der erwarteten Impedanz liegt, wobei mindestens einer des ersten und des zweiten Spannungsimpulses an die Last therapeutisch ist, nicht abzugeben.

2. System (100, 2150) nach Anspruch 1, wobei die erwartete Impedanz der Last durch das System (100, 2150) basierend auf einem Typ der verwendeten Elektrode bestimmt wird oder die erwartete Impedanz hochgeladen, eingegeben oder in das System programmiert wird.

3. System (100, 2150) nach Anspruch 1, wobei die Steuerung (2175) ferner konfiguriert ist, um die erwartete Impedanz basierend auf einer Identifikation der Last zu bestimmen, wobei optional die erwartete Impedanz einen Impedanzbereich umfasst.

4. System (100, 2150) nach einem der Ansprüche 1 bis 3, wobei die Steuerung (2175) konfiguriert ist, um den Test mehrmals durchzuführen, und wobei das Bestimmen, den dritten Spannungsimpuls zu liefern, zumindest teilweise auf dem Bestehen des Tests für mindestens eine Anzahl aufeinanderfolgender Zeiten basiert.

5. System (100, 2150) nach Anspruch 1, wobei die Steuerung (2175) konfiguriert ist, um den Test mehrmals durchzuführen und den Test mindestens teilweise basierend auf einer Menge an Impedanzmessungen, die durchgeführt werden, zu bestimmen.

6. System (100, 2150) nach einem der Ansprüche 1 bis 3, wobei die Steuerung (2175) konfiguriert ist, um den Test mehrmals durchzuführen und den Test mindestens teilweise basierend auf einem Zeitgrenzzustand zu bestimmen.

7. System (100, 2150) nach einem der Ansprüche 1 bis 6, wobei das System (100, 2150) konfiguriert ist, um die Impulsgeneratorschaltung (2155) auf eine dritte Ladespannung zu laden oder zu entladen.

8. System (100, 2150) nach einem der Ansprüche 1 bis 7, wobei das System (100, 2150) konfiguriert ist, um eine grafische Anzeige basierend auf dem Vergleich anzuzeigen.

9. System (100, 2150) nach einem der Ansprüche 1 bis 8, wobei der zweite Spannungsimpuls ein Niederspannungstestimpuls ist und der erste Spannungsimpuls ein therapeutischer Behandlungsimpuls ist.

10. System (100, 2150) nach einem der Ansprüche 1 bis 8, ferner umfassend eine Niederspannungsquelle.

11. System (100, 2150) nach einem der Ansprüche 1 bis 10, wobei die Impedanz der Last bestimmt wird durch:
Messen eines Stroms, wobei der Strom während der Abgabe des zweiten Spannungsimpulses an die Last abgegeben wird;
Bestimmen einer Spannung des abgegebenen zweiten Spannungsimpulses; und Berechnen der Impedanz basierend auf einem Spannungspegel des zweiten Spannungsimpulses und auf dem gemessenen Strom.

12. System (100, 2150) nach einem der Ansprüche 1 bis 11, wobei die Steuerung (2175) ferner konfiguriert ist, um die Impulsgeneratorschaltung (2155) zu veranlassen, einen dritten Spannungsimpuls an die Last abzugeben, und wobei das Abgeben des dritten Spannungsimpulses das Ändern eines Zustands eines Schalters umfasst, um die Last elektrisch von einer ersten Spannungsimpulsquelle zu trennen und die Last mit einer zweiten Spannungsimpulsquelle zu verbinden.

13. System (100, 2150) nach einem der Ansprüche 1 bis 12, wobei die Steuerung ferner konfiguriert ist, um zu bestimmen, ob ein Impedanztest abgeschlossen ist; und basierend auf dem Ergebnis von 1) eine Bestimmung, ob der Impedanztest abgeschlossen ist, und 2) der Vergleich der Impedanz mit der erwarteten Impedanz, bestimmen, ob ein Impuls eines Testspannungspegels oder eines Impulses eines therapeutischen Spannungsniveaus angewendet oder das Anlegen von Impulsen zu beendet werden soll.

14. System (100, 2150) nach einem der Ansprüche 1 bis 13, wobei die Steuerung (2175) ferner konfiguriert ist, um die Impulsgeneratorschaltung (2155) zu veranlassen, einen dritten Spannungsimpuls an die Last abzugeben, und wobei der dritte Spannungsimpuls ein Behandlungsimpuls ist.

15. Therapeutisches nsPEF-Impulsgeneratorsystem (100, 2150), umfassend:
eine Elektrode (2165); eine Impulsgeneratorschaltung (2155), die elektrisch mit der Elektrode (2165) verbunden und konfiguriert ist, um Spannungsimpulse an die Elektrode (2165) abzugeben; und eine Steuerung (2175), die mit der Impulsgeneratorschaltung (2155) verbunden und konfiguriert ist, um einen Spannungspegel der Spannungsimpulse zu bestimmen, die an die Elektrode abgegeben werden, wobei die Steuerung (2175) ferner konfiguriert ist zum:
Veranlassen der Impulsgeneratorschaltung (2155), einen ersten Spannungsimpuls an eine Last durch die Elektrode abzugeben;
Bestimmen einer ersten Impedanz der Last basierend mindestens teilweise auf dem abgegebenen ersten Spannungsimpuls;
Veranlassen der Impulsgeneratorschaltung (2155), einen zweiten Spannungsimpuls an eine Last durch die Elektrode abzugeben;
Bestimmen einer zweiten Impedanz der Last basierend mindestens teilweise auf dem abgegebenen zweiten Spannungsimpulssignal;
Vergleichen der ersten Impedanz mit der zweiten Impedanz; und
als Ergebnis des Vergleichs, Entladen der Impulsgeneratorschaltung (2155) auf eine zweite Ladespannung, die niedriger als die erste Ladespannung ist, unter Verwendung einer Entladeschaltung zum:
a) Beenden der Abgabe von Spannungsimpulsen an die Last oder
b) Abgeben eines nächsten Spannungsimpuls an die Last, wobei der nächste Spannungsimpuls einen elektrischen Parameter aufweist, der sich von einem entsprechenden elektrischen Parameter des ersten und des zweiten Spannungsimpulses unterscheidet.

16. System (100, 2150) nach Anspruch 15, wobei die Steuerung (2175) konfiguriert ist, um die Abgabe von Spannungsimpulsen an die Last als Ergebnis einer Differenz, die größer oder kleiner als ein Schwellenwert ist oder außerhalb eines Schwellenbereichs liegt, zwischen der ersten und der zweiten Impedanz zu beenden.

17. System (100, 2150) nach Anspruch 15 oder 16, wobei die ersten und die zweiten Spannungsimpulse therapeutische Impulse sind oder wobei die ersten und die zweiten Spannungsimpulse Testimpulse sind.

18. System (100, 2150) nach einem der Ansprüche 15 bis 17, wobei der Spannungsimpuls, der den unterschiedlichen elektrischen Parameter aufweist, als Ergebnis des Vergleichs, der angibt, dass eine Abweichung von einem Impedanzprofil größer als ein Schwellenwert ist, abgegeben wird.

19. System (100, 2150) nach einem der Ansprüche 15 bis 18, wobei der elektrische Parameter einer von einer Spannung, einer Frequenz, einer Dauer und einer Spannungsform ist.

## Revendications

1. Système de générateur d'impulsions nsPEF thérapeutique (100, 2150), comprenant :
une électrode (2165) ; un circuit générateur d'impulsions (2155) connecté électriquement à l'électrode et configuré pour fournir des impulsions à l'électrode ; et un dispositif de commande (2175) connecté au circuit générateur d'impulsions (2155) et configuré pour déterminer un niveau de tension des impulsions fournies à l'électrode, dans lequel le dispositif de commande est en outre configuré pour :
amener le circuit générateur d'impulsions (2155) à être chargé à une première tension de charge,
amener le circuit générateur d'impulsions (2155) à fournir une première impulsion de tension à une charge au niveau de la première tension de charge,
amener le générateur d'impulsions (2155) à être déchargé à une deuxième tension de charge inférieure à la première tension de charge à l'aide d'un circuit de décharge,
amener le générateur d'impulsions (2155) à fournir une deuxième impulsion de tension à la charge à travers l'électrode (2165) au niveau de la deuxième tension de charge, recevoir un signal indiquant une impédance de la charge, comparer l'impédance avec une impédance attendue, et au moins partiellement en réponse à la comparaison, déterminer
1) de fournir une troisième impulsion de tension à la charge, au moins partiellement en fonction d'une détermination du fait que le circuit générateur d'impulsions (2155) a passé un test en fonction au moins partiellement de l'impédance se trouvant dans une plage seuil de l'impédance attendue ; et
2) de ne pas fournir une troisième impulsion de tension à la charge au moins partiellement en fonction d'une détermination du fait que l'impédance se trouve en dehors de la plage seuil de l'impédance attendue, dans lequel au moins l'une des première et deuxième impulsions de tension est thérapeutique à la charge.

2. Système (100, 2150) selon la revendication 1, dans lequel l'impédance attendue de la charge est déterminée par le système (100, 2150) en fonction d'un type d'électrode utilisé, ou l'impédance attendue est chargée, entrée ou programmée dans le système.

3. Système (100, 2150) selon la revendication 1, dans lequel le dispositif de commande (2175) est en outre configuré pour déterminer l'impédance attendue en fonction d'une identification de la charge, éventuellement dans lequel l'impédance attendue comprend une plage d'impédance.

4. Système (100, 2150) selon l'une quelconque des revendications 1 à 3, dans lequel le dispositif de commande (2175) est configuré pour réaliser le test plusieurs fois, et dans lequel la détermination de fournir la troisième impulsion de tension est au moins partiellement en fonction du passage du test au moins un nombre de fois consécutives.

5. Système (100, 2150) selon la revendication 1, dans lequel le dispositif de commande (2175) est configuré pour réaliser le test plusieurs fois et déterminer que le test est terminé au moins partiellement en fonction d'une quantité de mesures d'impédance prises.

6. Système (100, 2150) selon l'une quelconque des revendications 1 à 3, dans lequel le dispositif de commande (2175) est configuré pour réaliser le test plusieurs fois et déterminer que le test est terminé au moins partiellement en fonction d'une condition de limite de temps.

7. Système (100, 2150) selon l'une quelconque des revendications 1 à 6, dans lequel le système (100, 2150) est configuré pour charger ou décharger le circuit générateur d'impulsions (2155) à une troisième tension de charge.

8. Système (100, 2150) selon l'une quelconque des revendications 1 à 7, dans lequel le système (100, 2150) est configuré pour afficher une indication graphique en fonction de la comparaison.

9. Système (100, 2150) selon l'une quelconque des revendications 1 à 8, dans lequel la deuxième impulsion de tension est une impulsion de test basse tension et la première impulsion de tension est une impulsion de traitement thérapeutique.

10. Système (100, 2150) selon l'une quelconque des revendications 1 à 8, comprenant en outre une source de basse tension.

11. Système (100, 2150) selon l'une quelconque des revendications 1 à 10, dans lequel l'impédance de la charge est déterminée par :
la mesure d'un courant, dans lequel le courant est fourni à la charge pendant la fourniture de la deuxième impulsion de tension ;
déterminer une tension de la deuxième impulsion de tension fournie ; et le calcul de l'impédance en fonction d'un niveau de tension de la deuxième impulsion de tension et du courant mesuré.

12. Système (100, 2150) selon l'une quelconque des revendications 1 à 11, dans lequel le dispositif de commande (2175) est en outre configuré pour amener le circuit générateur d'impulsions (2155) à fournir une troisième impulsion de tension à la charge et dans lequel la fourniture de la troisième impulsion de tension comprend le changement d'un état d'un commutateur de manière à déconnecter électriquement la charge d'une première source d'impulsion de tension et à connecter la charge à une deuxième source d'impulsion de tension.

13. Système (100, 2150) selon l'une quelconque des revendications 1 à 12, dans lequel le dispositif de commande est en outre configuré pour déterminer si un test d'impédance est terminé ; et en fonction du résultat 1) d'une détermination du fait de savoir si le test d'impédance est terminé et 2) de la comparaison de l'impédance avec l'impédance attendue, déterminer s'il faut appliquer une impulsion d'un niveau de tension de test ou une impulsion d'un niveau de tension thérapeutique ou arrêter d'appliquer des impulsions.

14. Système (100, 2150) selon l'une quelconque des revendications 1 à 13, dans lequel le dispositif de commande (2175) est en outre configuré pour amener le circuit générateur d'impulsions (2155) à fournir une troisième impulsion de tension à la charge et dans lequel une troisième impulsion de tension est une impulsion de traitement.

15. Système de générateur d'impulsions nsPEF thérapeutique (100, 2150), comprenant :
une électrode (2165) ; un circuit générateur d'impulsions (2155) connecté électriquement à l'électrode (2165) et configuré pour fournir des impulsions de tension à l'électrode (2165) ; et un dispositif de commande (2175) connecté au circuit générateur d'impulsions (2155) et configuré pour déterminer un niveau de tension des impulsions de tension fournies à l'électrode, dans lequel le dispositif de commande (2175) est en outre configuré pour :
amener le circuit générateur d'impulsions (2155) à fournir une première impulsion de tension à une charge à travers l'électrode ;
déterminer une première impédance de la charge au moins partiellement en fonction de la première impulsion de tension fournie ;
amener le circuit générateur d'impulsions (2155) à fournir une deuxième impulsion de tension à une charge à travers l'électrode ;
déterminer une seconde impédance de la charge au moins partiellement en fonction de la deuxième impulsion de tension fournie ; comparer la première impédance à la seconde impédance ; et en conséquence de la comparaison, la décharge du circuit générateur d'impulsions (2155) à une deuxième tension de charge inférieure à la première tension de charge à l'aide d'un circuit de décharge pour :
a) arrêter la fourniture d'impulsions de tension à la charge, ou
b) fournir une impulsion de tension suivante à la charge, dans lequel l'impulsion de tension suivante a un paramètre électrique qui est différent d'un paramètre électrique correspondant des première et deuxième impulsions de tension.

16. Système (100, 2150) selon la revendication 15, dans lequel le dispositif de commande (2175) est configuré pour arrêter la fourniture d'impulsions de tension à la charge en raison d'une différence entre la première et la seconde impédance étant supérieure ou inférieure à un seuil ou se trouvant en dehors d'une plage seuil.

17. Système (100, 2150) selon les revendications 15 ou 16, dans lequel les première et deuxième impulsions de tension sont des impulsions thérapeutiques, ou dans lequel les première et deuxième impulsions de tension sont des impulsions de test.

18. Système (100, 2150) selon l'une quelconque des revendications 15 à 17, dans lequel l'impulsion de tension ayant le paramètre électrique différent est fournie en conséquence de la comparaison indiquant un écart par rapport à un profil d'impédance étant supérieur à un seuil.

19. Système (100, 2150) selon l'une quelconque des revendications 15 à 18, dans lequel le paramètre électrique est l'un parmi une tension, une fréquence, une durée et une forme de tension.
